# EUROPEAN PATENT SPECIFICATION

(11) **EP 4 003 415 B1**
(45) Date of publication and mention of the grant of the patent: **11.09.2024**
(21) Application number: 20743142.0
(22) Date of filing: 22.07.2020
(51) Int. Cl.: A61K 39/395, A61K 38/17, A61P 35/00, G01N 33/50

(54) **INHIBITORS OF CHI3L1 AND THEIR USES**
INHIBITOREN VON CHI3L1 UND IHRE VERWENDUNGEN
INHIBITEURS DE CHI3L1 ET LEURS UTILISATIONS

(30) Priority: 22.07.2019 IT 201900012540
(43) Date of publication of application: 01.06.2022
(73) Proprietor: Humanitas Mirasole S.p.A., 20100 Rozzano (MI) (IT); Humanitas University, 20090 Pieve Emanuele (MI) (IT); Istituto Europeo di Oncologia S.r.l., 20121 Milano (MI) (IT)
(72) Inventor: PENNA, Giuseppe, 20100 Rozzano (MI) (IT); RESCIGNO, MAria, 20100 Rozzano (MI) (IT); DARWICH, Abbass, 20100 Rozzano (MI) (IT)
(74) Representative: Currado, Luisa
(86) International application number: PCT/EP2020/070665
(87) International publication number: WO 2021/013884

(56) References cited:
- WO-A1-2010/028658
- WO-A1-2019/040685
- DO-HYUN KIM ET AL: "Regulation of chitinase-3-like-1 in T cell elicits Th1 and cytotoxic responses to inhibit lung metastasis", NATURE COMMUNICATIONS, vol. 9, no. 1, 5 February 2018 (2018-02-05), XP055624173, DOI: 10.1038/s41467-017-02731-6
- DO-HYUN KIM ET AL: "Chitinase 3-like-1, a novel regulator of Th1/CTL responses, as a therapeutic target for increasing anti-tumor immunity", BMB REPORTS, vol. 51, no. 5, 31 May 2018 (2018-05-31), pages 207 - 208, XP055663636, DOI: 10.5483/BMBRep.2018.51.5.094
- LINE S. TARPGAARD ET AL: "Plasma YKL-40 in Patients with Metastatic Colorectal Cancer Treated with First Line Oxaliplatin-Based Regimen with or without Cetuximab: RESULTS from the NORDIC VII Study", PLOS ONE, vol. 9, no. 2, 3 February 2014 (2014-02-03), pages e87746, XP055663789, DOI: 10.1371/journal.pone.0087746
- JOHANSEN JULIA S ET AL: "Is YKL-40 a new therapeutic target in cancer?", EXPERT OPINION ON THERAPEUTIC TARGETS, INFORMA HEALTHCARE, UK, vol. 11, no. 2, 1 February 2007 (2007-02-01), pages 219 - 234, XP008125745, ISSN: 1472-8222, DOI: 10.1517/14728222.11.2.219

## Description

### TECHNICAL FIELD

The present invention relates to a suppressor or inhibitor of the expression and/or the activity of Chitinase 3-like 1 (CHI3L1) for use in the prevention and/or treatment of tumors, wherein said tumors are resistant to antibody-dependent cell-mediated cytotoxicity (ADCC) dependent therapies.

### BACKGROUND ART

Trastuzumab and Pertuzumab target the human epidermal growth factor receptor-2 (HER2) which is overexpressed in approximately 20% of breast cancers and is associated with poor clinical prognosis and patient survival. Despite the good improvement in progression free survival obtained by combining these antibodies, half the patients still progress in less than two years. This highlights the necessity to understand how resistance is occurring despite efficient blockade of HER2 signaling by the two antibodies.

Do-Hyun Kim et al. (Nat Commun. 2018 Feb 5;9(1):503. Regulation of chitinase-3-like-1 in T cell elicits Th1 and cytotoxic responses to inhibit lung metastasis) discloses silencing of CHI3L1 expression to inhibit lung metastasis.

Do-Hyun Kim and Je-Min Choi (BMB Rep. 2018 May;51(5):207-208. Chitinase 3-like-1, a novel regulator of Th1/CTL responses, as a therapeutic target for increasing anti-tumor immunity) disclose that targeted knockdown by Chi3l1 siRNA complexed with the cell-penetrating peptide dNP2 showed decreased pulmonary melanoma metastasis with increased infiltration of Th1 and CTL in the lung.

WO2019040685 discloses a method of treating cancer comprising administering an inhibitor of CHI3L1 and an inhibitor of an immune checkpoint protein. WO2011127297 refers to methods of treating cancer, especially breast cancer, and in particular HER2/ErbB2 positive breast cancer using a FoxMl inhibitor in conjunction with trastuzumab and/or paclitaxel, pharmaceutical compositions comprising a FoxMl inhibitor in the presence of trastuzumab and/or paclitaxel and to methods of identifying and treating trastuzumab resistant and/or paclitaxel resistant cancer and of promoting breast tumor cell differentiation. Since FOXM1 is a transcription factor, its targeting is challenging, especially in vivo. Moreover, inhibition of FoxM1 has been shown to hinder healing process such as in liver wounding(Ren *et al.,* 2010). Further, FOXM1 exist in 4 isoforms and it is not clear how interaction and isoform switch occur between FOXM1a and FOXM1b or FOXM1c or FOXM1d.

Therefore, it is still felt the need of a therapeutic agent able to treat patients who do not respond or are resistant to common therapies.

### SUMMARY OF THE INVENTION

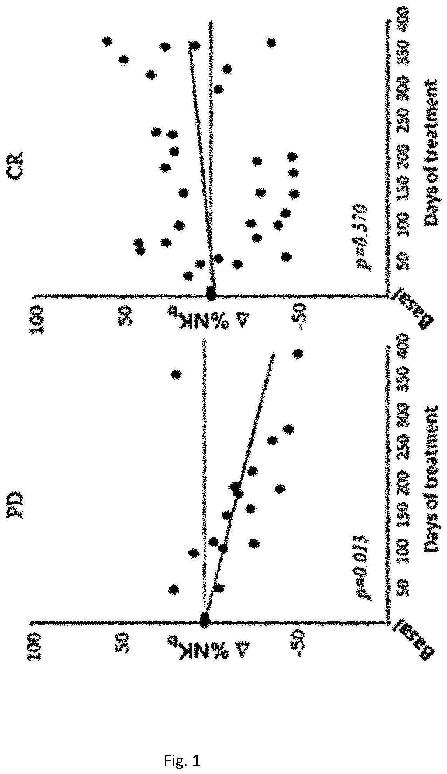

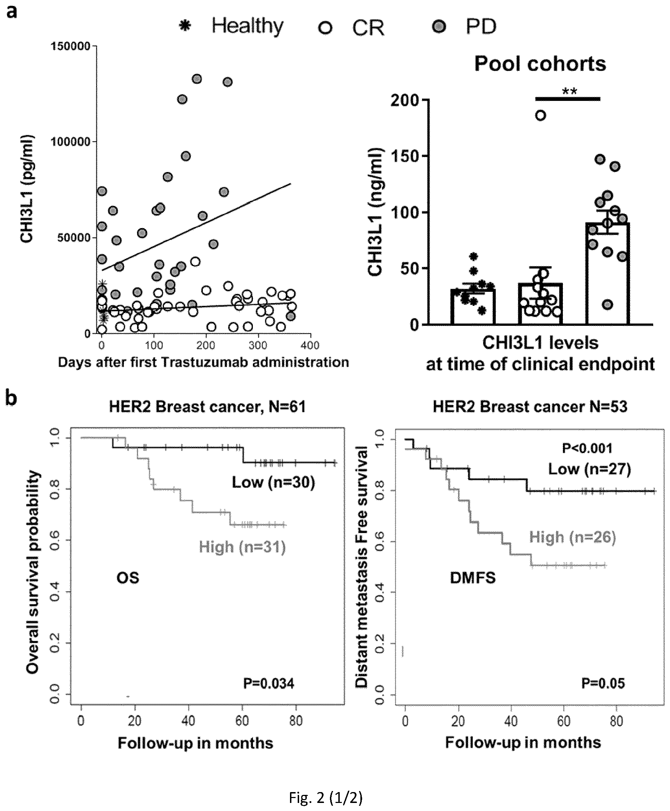

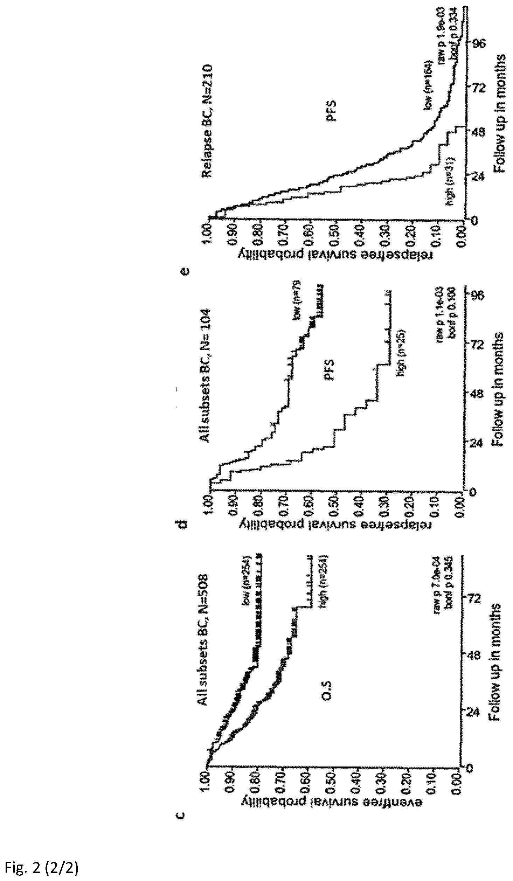

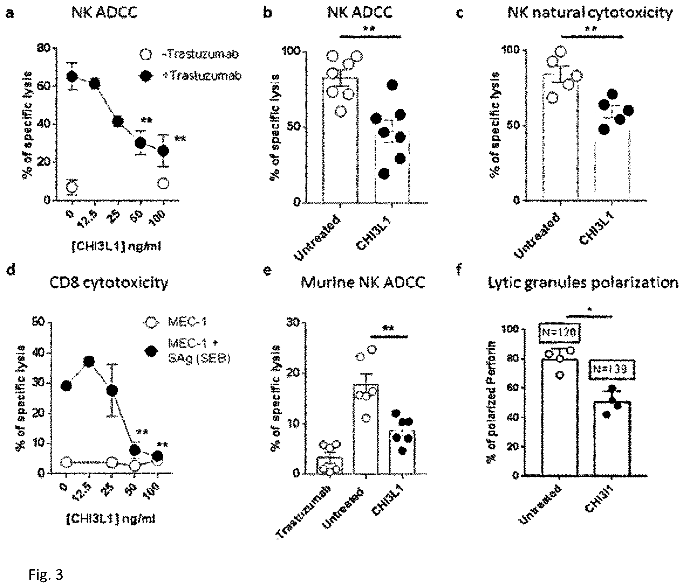

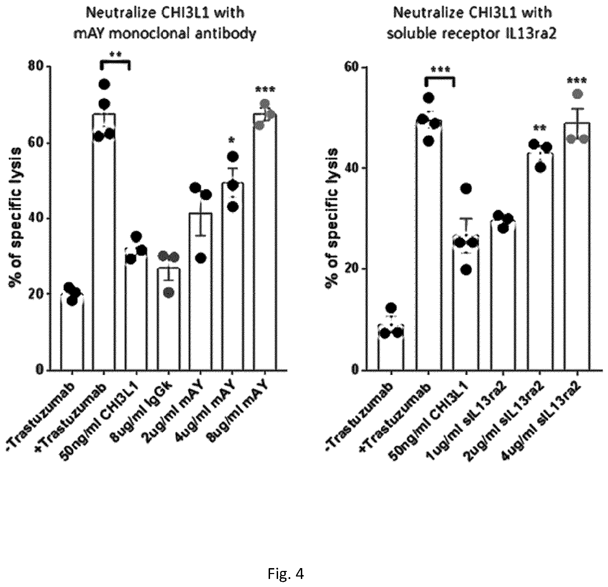

A major mechanism by which the above mentioned anti-HER2 antibodies act, is by additively enhancing antibody-dependent cytotoxicity (ADCC) by NK cells. Hence, inventors hypothesized that an impairment of NK cell ADCC could be a potential mechanism of resistance to HER2 targeted therapy. To test this, inventors obtained sera from HER2+ breast cancer patients undergoing trastuzumab adjuvant therapy at baseline and during treatment. NK cells from healthy donors were then exposed to sera from complete remission (CR) or progressive disease (PD) patients and their ADCC activity measured. A proteomic analysis of sera from the two groups of patients (PD vs RC) was then carried to reveal differentially expressed molecules. Inventors observed that disease progression to trastuzumab treatment was associated with the capacity of sera from progressive disease (PD) patients to inhibit healthy NK cell ADCC (fig1). Inventors found the protein Chitinase 3-like 1 (CHI3L1) to be at high levels in sera of PD patients, compared to CR patients and healthy controls (fig2), and then showed that recombinant CHI3L1 protein directly inhibits trastuzumab mediated NK cells ADCC in vitro (fig3). Finally, inventors could reverse this inhibition by adding two different neutralizing ligands of CHI3L1, a specific monoclonal antibody and the soluble form of its receptor interleukin 13 receptor alpha 2 (IL13ra2) (fig4).

### DETAILED DESCRIPTION OF THE INVENTION

The invention is defined in the claims.

The present invention relates to ligands, such as monoclonal antibodies or engineered soluble receptors of CHI3L1/YKL-40, which are capable of restoring ADCC activity of NK cells by neutralizing CHI3L1/YKL40 activity, for the treatment of a disease wherein ADCC activity of NK cells is prerequisite for successful therapy. For example, in combination with Trastuzumab to prevent or overcome treatment resistance in Her2 breast cancer.

Inventors show that CHI3L1 is present at high levels in sera of patients that resist to Trastuzumab. The sera from these patients inhibit NK cell ADCC activity which has been shown to be detrimental for trastuzumab activity in patients. Inventors make the link by showing a new role of CHI3L1 in directly inhibiting NK cells ADCC. This is a potential mechanism of resistance to HER2 targeted therapy but also to any ADCC dependent therapy. Combination of Trastuzumab/Pertuzumab and CHI3L1 targeting ligands may be a new approach to avoid or overcome resistance.

The present results regarding Trastuzumab resistance in breast cancer are plausible for any malignancy given that:
(1) CHI3L1 is expressed at high levels in multiple cancer types including lung, prostate, gastric, colon, rectum, ovary, kidney, liver, breast, glioblastomas, and malignant melanoma among others (Eurich *et al.,* 2009; Johansen, Schultz and Jensen, 2009; Choi *et al.,* 2010; Francescone *et al.,* 2011; Zhang *et al.,* 2014; Erturk *et al.,* 2017; Qiu *et al.,* 2018; Lin *et al.,* 2019. (Libreros S, Iragavarapu-Charyulu V. YKL-40/CHI3L1 drives inflammation on the road of tumor progression. J Leukoc Biol. 2015;98(6):931-936),
(2) Tumors and/or metastasis are treated with a monoclonal antibody (e.g. those of table 1 or those cited in ⁷) that functions, at least in part, by mediating NK cell ADCC,
(3) These tumors and/or metastasis are not responding or just partially responding to treatment with such antibody.

It is therefore an object of the present invention a suppressor or inhibitor of the expression and/or the activity of Chitinase 3-like 1 (CHI3L1) for use in the prevention and/or treatment of tumors, wherein said tumors are resistant to antibody-dependent cell-mediated cytotoxicity (ADCC) dependent therapies
wherein the tumor comprises HER2 positive cancer cells and is resistant to anti-HER2 antibody therapies, preferably it is resistant to Trastuzumab and/or to Pertuzumab, and wherein the suppressor or inhibitor is at least one molecule selected from the group consisting of: an anti-CHI3L1 antibody or a fragment thereof, and a host cell genetically engineered expressing said antibody or the soluble form of CHI3L1 receptor, wherein said antibody-dependent cell-mediated cytotoxicity (ADCC) is of NK cells.

It is a further object of the invention a suppressor or inhibitor of the expression and/or the activity of Chitinase 3-like 1 (CHI3L1) for use in the prevention and/or treatment of tumors, wherein said tumors are resistant to antibody-dependent cell-mediated cytotoxicity (ADCC) dependent therapies, wherein the tumor is a tumor which is resistant to Trastuzumab, wherein the suppressor or inhibitor is at least one molecule selected from the group consisting of: an anti-CHI3L1 antibody or a fragment thereof, and a host cell genetically engineered expressing said antibody or the soluble form of CHI3L1 receptor, wherein said antibody-dependent cell-mediated cytotoxicity (ADCC) is of NK cells.

Preferably CHI3L1 is extracellular, more preferably tumor-derived. Preferably the tumor expresses CHI3L1 at high levels. In particular, the tumor may express CHI3L1 at high levels compared to adjacent healthy tissues and/or CHI3L1 may be detected at elevated levels in a serum/plasma sample of the patient compared to the reference level in healthy population (~30ng/ml), preferably CHI3L1 may be above 50ng/ml, more preferably above 80ng/ml. Preferably the tumor is a lung, prostate, gastric, colon, rectum, ovary, kidney, liver, breast, glioblastomas, and malignant melanoma, more preferably the tumor is HER2+ breast cancer and HER2+ gastric cancer such as metastatic gastric adenocarcinoma.

Preferably, the tumor is a breast tumor comprising HER2 positive cancer cells that is resistant to anti-HER2 therapies, more preferably it is resistant to Trastuzumab and/or to Pertuzumab.

The tumor comprises HER2 positive cancer cells, preferably being a breast tumor or a gastric cancer such as a metastatic gastric adenocarcinoma, and is resistant to anti-HER2 antibody therapies, preferably it is resistant to Trastuzumab and/or to Pertuzumab.

Said suppressor or inhibitor is preferably capable of restoring antibody-dependent cell-mediated cytotoxicity (ADCC) of NK cells.

Preferably, said suppressor or inhibitor is an anti-CHI3L1 antibody, more preferably a monoclonal antibody, even more preferably the clone mAY, or an inhibitor of CHI3L1 receptor, or the soluble form of CHI3L1 receptor, preferably interleukin 13 receptor alpha 2, or Receptor for Advance Glycation End product (RAGE).

Examples of inhibitors of CHI3L1 receptor are:
- Inhibitors of RAGE-ligand interaction or RAGE-signaling: small molecule inhibitors (e.g Azeliragon), recombinant soluble RAGE (sRAGE), inhibitors of cytoplasmic domain ctRAGE interaction with Diaphanus-1 (DIAPH-1) signaling molecule, RAGE antagonistic peptides, RAGE blocking antibodies, RAGE specific CAR T cells or CAR NK cells.
- Inhibitors of IL13ra2: recombinant soluble IL13Ra2 (sIL13Ra2), IL13ra2 blocking antibodies, Il13Ra2 specific CAR-T or CAR NK cells.

Preferably the tumor is resistant to Trastuzumab.

In a preferred embodiment, the suppressor or inhibitor is used in combination with at least one therapeutic antibody which is capable of antibody-dependent cell-mediated cytotoxicity (ADCC), e.g. an anti-HER2, an anti-CD20, an anti-GD20, an anti-CCR4, an anti-EGFR, an anti-CD 19 or an anti-MUC1 or an anti-GA201 antibody, more preferably with Trastuzumab, Pertuzumab, Rituximab, Dinituximab, Obinutuzumab, Mogamulizumab, Panitumumab and/or Cetuximab, more preferably Trastuzumab and/or Pertuzumab.

Another object of the invention is a pharmaceutical composition comprising:
i. at least one suppressor or inhibitor of expression and/or activity of Chitinase 3-like 1 (CHI3L1) as defined above,
ii. at least one therapeutic antibody which is capable of antibody-dependent cell-mediated cytotoxicity (ADCC) and
iii. at least one pharmaceutically acceptable excipient, diluent or carrier for use in the prevention and/or treatment of tumors,
wherein said tumors are resistant to antibody-dependent cell-mediated cytotoxicity (ADCC) dependent therapies, wherein the tumor comprises HER2 positive cancer cells, preferably a breast tumor or a gastric cancer, and is resistant to anti-HER2 antibody therapies, preferably it is resistant to Trastuzumab and/or to Pertuzumab.

Preferably said antibody which is capable of antibody-dependent cell-mediated cytotoxicity (ADCC) is at least one antibody anti-HER2, an anti-CD20, an anti-GD20, an anti-CCR4, an anti-EGFR, an anti-CD19 or an anti-MUC1 or an anti-GA201, more preferably it is at least one antibody selected from the group consisting of: Trastuzumab, Pertuzumab, Rituximab, Dinituximab, Obinutuzumab, Mogamulizumab, Panitumumab and/or Cetuximab, more preferably Trastuzumab and/or Pertuzumab.

Said pharmaceutical composition preferably further comprises a therapeutic agent, wherein said therapeutic agent is for the treatment and/or prevention of tumours.

More preferably the tumor is a breast tumor comprising HER2 positive cancer cells that is resistant to anti-HER2 antibody therapies, preferably it is resistant to Trastuzumab and/or to Pertuzumab.

The tumor comprises HER2 positive cancer cells, preferably a breast tumor or a gastric cancer such as a metastatic gastric adenocarcinoma, and is resistant to anti-HER2 antibody therapies, preferably it is resistant to Trastuzumab and/or to Pertuzumab.

It is also disclosed an in vitro method, not encompassed by the wording of the claims, for identifying patient with a tumor which is resistant to antibody-dependent cell-mediated cytotoxicity (ADCC) dependent therapies, comprising the steps of:
a) detecting CHI3L1 in an isolated biological sample obtained from the patient and
b) comparing with respect to a proper control,
wherein an amount of CHI3L1 in the isolated biological sample obtained from the subject higher than the control amount (or proper control) indicates that tumor is resistant to antibody-dependent cell-mediated cytotoxicity (ADCC) dependent therapies.

It is also disclosed an in vitro method, not encompassed by the wording of the claims, for prognosing and/or diagnosing and/or assessing the risk of developing and/or for monitoring the progression and/or for monitoring the efficacy of a therapeutic treatment and/or for the screening of a therapeutic treatment of a tumour resistant to antibody-dependent cell-mediated cytotoxicity (ADCC) dependent therapies in a subject comprising the steps of:
a) detecting CHI3L1 in an isolated biological sample obtained from the subject and
b) comparing with respect to a proper control.

The tumor comprises HER2 positive cancer cells, preferably a breast tumor or a gastric cancer such as a metastatic gastric adenocarcinoma, and is resistant to anti-HER2 antibody therapies, preferably it is resistant to Trastuzumab and/or to Pertuzumab

Preferably, when an amount of CHI3L1 in the isolated biological sample obtained from the subject is higher than the control amount or proper control, the subj ect has a poor prognosis and/or s/he is resistant to antibody-dependent cell-mediated cytotoxicity (ADCC) dependent therapies, e.g. Trastuzumab, Pertuzumab, Rituximab, Dinituximab, Obinutuzumab, Mogamulizumab, Panitumumab and/or Cetuximab.

Preferably the sample of step a) is obtained after a period of treatment. Preferably the proper control is a CHI3L1 level previously determined from the same subject.

Preferably when an amount of CHI3L1 in the isolated biological sample obtained from the subject is lower or equal than the proper control, it may indicate that the tested substance is effective for the treatment of the tumour.

Preferably when an amount of CHI3L1 in the isolated biological sample obtained from the subject is above the proper control, it indicates that the patient may be not responsive said treatment.

Preferably, when an amount of CHI3L1 in the isolated biological sample obtained from the subject is similar or lower than the proper control, the subject may be not affected by a tumour resistant to antibody-dependent cell-mediated cytotoxicity (ADCC) dependent therapies or go toward an amelioration of said tumour, respectively.

Preferably, when an amount of CHI3L1 in the isolated biological sample obtained from the subject is higher than the proper control, the subject may be affected by a tumour resistant to antibody-dependent cell-mediated cytotoxicity (ADCC) dependent therapies or go toward an worsening of said tumour.

Preferably, when an amount of CHI3L1 in the isolated biological sample obtained from the subject is higher than the proper control, the subject may be at risk of developing a tumour resistant to antibody-dependent cell-mediated cytotoxicity (ADCC) dependent therapies.

In the above method, preferably step a) comprises measuring the amount of the CHI3L1 or of fragments thereof or of the polynucleotide coding for said protein (DNA or mRNA) or of fragments thereof in said isolated biological sample obtained from the subject and step b) comprises comparing the measured amount of step a) with a proper control amount.

It is also disclosed a kit, not encompassed by the wording of the claims, for carrying out the above methods, comprising
- means to measure the amount or the activity of CHI3L1 or of fragments thereof and/or means to measure the amount of the polynucleotide coding for said protein or of fragments thereof and optionally,
- control means.

Control means can be used to compare the amount or the increase of amount of CHI3L1 to a proper control. The proper control may be obtained for example, with reference to known standard, either from a normal subject or from normal population, or from a subject presenting a tumor that is not resistant to antibody-dependent cell-mediated cytotoxicity (ADCC) dependent therapies.

The means to measure the amount of CHI3L1 as above defined are preferably at least one antibody, functional analogous or derivatives thereof. Said antibody, functional analogous or derivatives thereof are specific for said marker.

In a preferred embodiment, the kit of the invention comprises:
- a solid phase adhered antibody specific for said compound;
- detection means of the ligand specific-marker complex.

Alternatively, the reagents can be provided as a kit comprising reagents in a suspension or suspendable form, e.g. reagents bound to beads suitable for flow cytometry, preferably magnetic beads coated with antibody capture. The instructions may comprise instructions for conducting an antibody-based flow cytometry assay.

Detection means are preferably means able to detect and/or measure the amount of the described markers, e.g. means able to detect the complex antigen-antibody, as enzyme conjugated secondary antibodies, luminescent substrates, magnetic beads coated with antibody capture, customized dried antibody cocktails and/or columns with size filter cartridges and/or combined with specific antibody filter (SAF). Other means may be e.g. specific primers and probes for RT PCR.

In an embodiment, the method further comprises selecting a therapeutic regimen based on the analysis. In an embodiment, the method further comprises determining a treatment course for the subject based on the analysis. The kits according to the invention can further comprise customary auxiliaries, such as buffers, carriers, markers, etc. and/or instructions for use.

In the context of the present invention the term "detecting" may be intended also as "measuring the amount" or "measuring the alteration". In the case of a method or a kit for assessing the risk and/or diagnosing and/or prognosing of a tumour resistant to antibody-dependent cell-mediated cytotoxicity (ADCC) dependent therapies, the proper control may be a sample taken from a healthy patient or from a patient affected by another disorder or pathology or from a patient affected by a tumor which is not resistant to antibody-dependent cell-mediated cytotoxicity (ADCC) dependent therapies, and the proper control amount or activity may be the amount or activity of the same protein or polynucleotide measured in a sample taken from a healthy patient or from a patient affected by another disorder or pathology or from a patient affected by a tumor which is not resistant to antibody-dependent cell-mediated cytotoxicity (ADCC) dependent therapies.

In the case of a method or a kit for monitoring the progression of a tumour resistant to antibody-dependent cell-mediated cytotoxicity tumour resistant to antibody-dependent cell-mediated cytotoxicity (ADCC) dependent therapies, the progress of the cancer is monitored and the proper control may be a sample taken from the same subject at various times or from another patient, and the proper control amount or activity may by the amount or activity of the same protein or polynucleotide measured in a sample taken from the same subject at various times or from another patient.

In the above method, preferably step a) comprises measuring the amount of CHI3L1 or of fragments thereof or of the polynucleotide coding for said protein (DNA or mRNA) or of fragments thereof in said isolated biological sample obtained from the subject and step b) comprises comparing the measured amount of step a) with a proper control amount.

Preferably, the in vitro method for monitoring the progression and/or for monitoring the efficacy of a therapeutic treatment of a tumour resistant to antibody-dependent cell-mediated cytotoxicity (ADCC) dependent therapies, as above defined, comprises the steps of:
a) measuring the alteration of the amount or the alteration of the activity of CHI3L1 or of fragments thereof or of the polynucleotide coding for said protein or fragments thereof in said isolated biological sample obtained from the subject and
b) comparing the measured alteration of step a) with a proper control alteration.

In the case of a method or a kit for monitoring the efficacy of a therapeutic treatment, the proper control may by a sample taken from the same subject before initiation of the therapy or taken at various times during the course of the therapy and the proper control amount or activity may be the amount or activity of the same protein or polynucleotide measured in a sample taken from the same subject before initiation of the therapy or taken at various times during the course of the therapy.

In the case of a method or a kit for the screening of a therapeutic treatment, the proper control may be a sample taken from subjects without treatment and from subjects treated with a substance that is to be assayed or from subjects treated with a reference treatment and the proper control amount or activity may be the average of the amounts or activity of the same protein or polynucleotide measured in samples taken from subjects without treatment and from subjects treated with a substance that is to be assayed or from subjects treated with a reference treatment. In this case, if the amount or activity of CHI3L1 or polynucleotide thereof in the isolated biological sample obtained from the subject is lower or equal than the control amount or activity, it may indicate that the tested substance is effective for the treatment of the tumour.

The patient with a tumor which is resistant to antibody-dependent cell-mediated cytotoxicity (ADCC) dependent therapies, who was identified by the in vitro methods as above defined may be treated with the suppressor or inhibitor of the present invention.

In the context of the present invention a tumor that is resistant to antibody-dependent cell-mediated cytotoxicity (ADCC) dependent therapies is:
- a tumor comprising HER2 positive cancer cells, preferably a breast tumor or a gastric cancer such as a metastatic gastric adenocarcinoma, that is resistant to anti-HER2 antibody therapies. In the context of the present invention a tumor that is resistant to antibody-dependent cell-mediated cytotoxicity (ADCC) dependent therapies is preferably a tumor expressing CHI3L1 at high levels. In particular, the tumor may express CHI3L1 at high levels compared to adjacent healthy tissues and/or CHI3L1 may be detected at elevated levels in a serum/plasma sample of the patient compared to the reference level in healthy population (~30ng/ml), preferably CHI3L1 may be above 50ng/ml, more preferably above 80ng/ml.

The tumor that is resistant to antibody-dependent cell-mediated cytotoxicity (ADCC) dependent therapies is preferably a lung, prostate, gastric, colon, rectum, ovary, kidney, liver, breast, glioblastomas, and malignant melanoma, more preferably the tumor is HER2+ breast cancer and HER2+ gastric cancer such as metastatic gastric adenocarcinoma.

In the present invention, the expression "measuring the amount" can be intended as measuring the amount (or the activity) or concentration or level of the respective protein and/or mRNA thereof and/or DNA thereof, preferably semi-quantitative or quantitative. Measurement of a protein can be performed directly or indirectly. Direct measurement refers to the amount or concentration measure of the marker, based on a signal obtained directly from the protein, and which is directly correlated with the number of protein molecules present in the sample. This signal - which can also be referred to as intensity signal - can be obtained, for example, by measuring an intensity value of a chemical or physical property of the marker. Indirect measurements include the measurement obtained from a secondary component (e.g., a different component from the gene expression product) and a biological measurement system (e.g. the measurement of cellular responses, ligands, "tags" or enzymatic reaction products).

The term "amount", as used in the description refers but is not limited to the absolute or relative amount of proteins and/or mRNA thereof and/or DNA thereof, and any other value or parameter associated with the same or which may result from these. Such values or parameters comprise intensity values of the signal obtained from either physical or chemical properties of the protein, obtained by direct measurement, for example, intensity values in an immunoassay, mass spectroscopy or a nuclear magnetic resonance. Additionally, these values or parameters include those obtained by indirect measurement, for example, any of the measurement systems described herein. Methods of measuring mRNA and DNA in samples are known in the art. To measure nucleic acid levels, the cells in a test sample can be lysed, and the levels of mRNA in the lysates or in RNA purified or semi-purified from lysates can be measured by any variety of methods familiar to those in the art. Such methods include hybridization assays using detectably labeled DNA or RNA probes (i.e., Northern blotting) or quantitative or semi-quantitative RT-PCR methodologies using appropriate oligonucleotide primers. Alternatively, quantitative or semi-quantitative in situ hybridization assays can be carried out using, for example, tissue sections, or unlysed cell suspensions, and detectably labeled (e.g., fluorescent, or enzyme-labeled) DNA or RNA probes. Additional methods for quantifying mRNA include RNA protection assay (RPA), cDNA and oligonucleotide microarrays, representation difference analysis (RDA), differential display, EST sequence analysis, and serial analysis of gene expression (SAGE).

If by comparing the measured amount or activity of the CHI3L1 or of the polynucleotide coding for said protein with the amount or activity obtained from a control sample, the amount or the activity of said marker in the sample isolated from the subject corresponds to a higher value, the subject may present tumour resistant to antibody-dependent cell-mediated cytotoxicity (ADCC) dependent therapies or go towards an aggravation of said disease.

If by comparing the measured amount or activity of the above markers or of the polynucleotide coding for said protein with the amount or the activity obtained from a control sample, the amount or the activity of said marker in the sample isolated from the subject corresponds to a similar or lower value, the subject may be not affected by a tumour resistant to antibody-dependent cell-mediated cytotoxicity (ADCC) dependent therapies or go toward an amelioration of said tumour, respectively.

Alternatively, the expression "detecting" or "measuring the amount" is intended as measuring the alteration of the molecule. Said alteration can reflect an increase or a decrease in the amount or activity of the molecules as above defined. An increase of the protein or of the activity of CHI3L1 or of the polynucleotide coding for said marker can be correlated to an aggravation of cancer. A decrease of the protein or of the activity of CHI3L1 or of the polynucleotide coding for said protein can be correlated to an amelioration of cancer or to recovery of the subject.

The term "suppressor or inhibitor of expression and/or activity of chitinase 3-like 1 (CHI3L1)" as used herein refers to a chemical compound or biological molecule that reduces expression of Chitinase 3-like 1 or inhibits Chitinase 3-like 1 activity in a cell.

The suppressor or inhibitor of expression and/or activity of the Chitinase 3-like 1 (CHI3L1) may also be used for use in inducing antibody-dependent cell-mediated cytotoxicity (ADCC) in a subj ect.

In the context of the present invention, "Chitinase 3-like 1 (CHI3L1) activity" comprises e.g.:
- Inhibition of NK cell ADCC and natural cytotoxicity
- Impairment of correct Microtubules and lytic granules polarization to the immune synapse by binding and modulating the signaling of receptor of advanced glycation end products (RAGE)
- emptying of lytic granules not towards a target
- Inhibition of CD8 T cells cytotoxicity.

In the context of the present invention "restoring antibody-dependent cell-mediated cytotoxicity (ADCC) of NK cells" includes also a partial restore. Indeed also a partial restore may be clinically sufficient. In particular the expression "restoring antibody-dependent cell-mediated cytotoxicity (ADCC) of NK cells" includes a restoration (also partial) that will result in (or is sufficient to achieve) clinical amelioration of the patient which may comprise:
- Complete tumor and/or metastasis regression, and/or
- Partial tumor and/or metastasis regression and/or
- No regression but not further progression of tumors and/or metastasis and/or
- Prevention of the recurrence (relapse) of tumors and or/metastasis.

In the context of the present invention a sample is preferably a serum or plasma sample, preferably from a patient having breast cancer that is HER2 positive.

In the context of the present invention an antibody-dependent cell-mediated cytotoxicity (ADCC) dependent therapies or a therapeutic antibody which is capable of antibody-dependent cell-mediated cytotoxicity (ADCC) comprises ADCC-Enhancing Antibodies, preferably Approved or in Clinical Trials.

Preferably said antibodies are Trastuzumab, Pertuzumab, Rituximab, Dinituximab, Obinutuzumab, Mogamulizumab, Cetuximab and/or Panitumumab and/or other antibodies in clinical development or awaiting FDA approval, such as Margetuximab (targeting HER2), MOR208 (targeting CD19), Ublituximab (targeting CD20), MEDI-551 (targeting CD19), Gatipotuzumab (targeting MUC1), Tomuzotuximab (targetin EGFR), Ocaratuzumab (targeting CD20), RO5083945 (GA201) (targeting EGFR), TrasGEX (targeting HER2) (⁷ ) or combination thereof.

Therefore, in the present invention the tumor may be resistant to one or more of the above antibodies. The corresponding biosimilars are also included in the present invention.

The detection of CHI3L1 is preferably carried out using a reagent that specifically detects CHI3L1.

In the method according to the invention, an amount of CHI3L1 in the isolated biological sample obtained from the subject higher than the control amount indicates that the subject has a poor prognosis and/or that s/he is resistant to antibody-dependent cell-mediated cytotoxicity (ADCC) dependent therapies, e.g. Trastuzumab, Pertuzumab, Rituximab, Dinituximab, Obinutuzumab, Mogamulizumab, Panitumumab and/or Cetuximab.

Preferably, the biological sample is a fluid, a cell or a tissue sample, more preferably said sample is plasma or serum.

The term "biological sample" encompasses a clinical sample, and also includes tissue obtained by surgical resection, tissue obtained by biopsy, cells in culture, cell supernatants, cell lysates, tissue samples, organs, bone marrow, blood, plasma, serum, and the like. A "sample" in the context of the present teachings refers to any biological sample that is isolated from a subject. A sample can include, without limitation an aliquot of body fluid, whole blood, serum, plasma, solid tissue samples such as tissue biopsies, or tissue cultures or cells derived therefrom and the progeny thereof, synovial fluid, lymphatic fluid, ascites fluid, and interstitial or extracellular fluid. The term "sample" also encompasses the fluid in spaces between cells, including gingival crevicular fluid, bone marrow, cerebrospinal fluid (CSF), saliva, mucous, sputum, semen, sweat, urine, or any other bodily fluids. "Blood sample" can refer to whole blood or any fraction thereof, including serum and plasma. Samples can be obtained from a subject by means including but not limited to venipuncture, excretion, ejaculation, massage, biopsy, needle aspirate, lavage, scraping, surgical incision, or intervention or other means known in the art. The definition also includes samples that have been manipulated in any way after their procurement, such as by treatment with reagents; washed; or enrichment for certain cell populations, such as cancer cells or samples in which regulatory T cells, are isolated and then analyzed. The definition also includes samples that have been enriched for particular types of molecules, e.g., nucleic acids, polypeptides, etc.

It is also disclosed a kit, not encompassed by the wording of the claims, for carrying out the above methods, comprising
- means to measure the amount or the activity of CHI3L1 or of fragments thereof and/or means to measure the amount of the polynucleotide coding for said protein or of fragments thereof and optionally,
- control means.

The above method preferably further comprises the steps of obtaining a control sample from a subject and assaying the control sample to detect CHI3L1 expression therein, wherein the cancer in the patient is trastuzumab-resistant if CHI3L1 expression is greater in the patient's sample than CHI3L1 expression in the control sample.

Preferably, the above reagent comprises one or more CHI3L1-specific primers, and the level of CHI3L1 expression is determined by reverse-transcriptase polymerase chain reaction (RT-PCR). Alternatively, the above reagent may be a CHI3L1-specific antibody and the level of CHI3L1 expression is determined by an immunoassay.

In order to select patients that may be treated with the present pharmaceutic composition, the following steps may be carried out:
(a) obtaining a sample from a patient in need of the treatment;
(b) detecting CHI3L1 expression in the sample using a reagent that specifically detects CHI3L1;
(c) obtaining a control sample; and
(d) assaying the control sample to detect CHI3L1 expression therein, wherein a CHI3L1 inhibitor is administered to the patient with trastuzumab if CHI3L1 expression in the sample is greater than CHI3L1 expression in the control sample.

Control means can be used to compare the amount or the increase of amount of CHI3L1 to a proper control. The proper control may be obtained for example, with reference to known standard, either from a normal subject or from normal population, or from a subject affected by a tumor that is not resistant to antibody-dependent cell-mediated cytotoxicity (ADCC) dependent therapies.

The means to measure the amount of CHI3L1 as above defined are preferably at least one antibody, functional analogous or derivatives thereof. Said antibody, functional analogous or derivatives thereof are specific for said CHI3L1.

A "control sample" as the term is used herein can be a normal, noncancerous breast tissue sample obtained from a proximal or distal site of the breast tissue from a breast cancer patient, or a tissue sample from a tumour that is not resistant to antibody-dependent cell-mediated cytotoxicity (ADCC) dependent therapies. It can also be obtained from an individual that does not have breast cancer. Similarly, the term "control tissue sample" refers to a corresponding tissue sample from an individual that does not have cancer or a non-cancerous tissue sample from a proximal or distal site of the tissue from a cancer patient or a tumour sample that is nor resistant to antibody-dependent cell-mediated cytotoxicity (ADCC) dependent therapies

In the method according to the invention, an amount of CHI3L1 in the isolated biological sample obtained from the subject higher than the control amount indicates that the subject has a poor prognosis or has a tumour which is resistant to antibody-dependent cell-mediated cytotoxicity (ADCC) dependent therapies or is at risk of developing a cancer resistant to antibody-dependent cell-mediated cytotoxicity (ADCC) dependent therapies.

By the term "suppressor or inhibitor" it is meant a molecule that effects a change in the expression and/or function of the target. The change is relative to the normal or baseline level of expression and/or function in the absence of the suppressor or inhibitor, but otherwise under similar conditions, and it represents a decrease in the normal/baseline expression and/or function.

The suppression or inhibition of the expression and/or function of the target may be assessed by any means known to the skilled in the art. The assessment of the expression level or of the presence of the target is preferably performed using classical molecular biology techniques such as (real time Polymerase Chain Reaction) qPCR, microarrays, bead arrays, RNAse protection analysis or Northern blot analysis or cloning and sequencing.

The assessment of target function is preferably performed by in vitro suppression assay, whole transcriptome analysis, mass spectrometry analysis to identify proteins interacting with the target. In the context of the present invention, the target may be the gene, the mRNA, the cDNA, or the encoded protein thereof, including fragments, derivatives, variants, isoforms, etc. Preferably, the target (i.e. CHI3L1) is characterized by its Accession numbers and/or amino acid and nucleotide sequences, herein disclosed.

In the context of the present disclosure, the term "polynucleotide" includes DNA molecules (e.g., cDNA or genomic DNA) and RNA molecules (e.g., mRNA, siRNA, shRNA) and analogs of the DNA or RNA generated using nucleotide analogs. The polynucleotide may be single-stranded or double-stranded.

In the context of the present invention the term "CHI3L1" includes the polynucleotide (e.g. the gene or the transcript) and the polypeptide (or protein) thereof.

The term polynucleotide and polypeptide may also include derivatives and functional fragments thereof. The polynucleotide may be synthesized using oligonucleotide analogs or derivatives (e.g., inosine or phosphorothioate nucleotides).

The genes/proteins as above defined are preferably characterized by the sequences identified by their NCBI Gene ID and Gen Bank Accession numbers. The term gene herein may also include corresponding orthologous or homologous genes, isoforms, variants, allelic variants, functional derivatives, functional fragments thereof. The expression "protein" is intended to include also the corresponding protein encoded from a corresponding orthologous or homologous genes, functional mutants, functional derivatives, functional fragments or analogues, isoforms thereof.

In the context of the present disclosure, the term "polypeptide" or "protein" includes:
i. the whole protein, allelic variants and orthologs thereof;
ii. any synthetic, recombinant or proteolytic functional fragment;
iii. any functional equivalent, such as, for example, synthetic or recombinant functional analogues.

In the context of the present disclosure an "anti-CHI3L1 antibody" includes an antibody (or fragments or derivatives thereof) directed against and/or targeting and/or binding to and/or neutralizing human CHI3L1. The anti-CHI3L1 antibody is able to inhibit or suppress (partially or completely) the expression and/or the activity of Chitinase 3-like 1.

The above defined antibodies comprise human and animal monoclonal antibodies or fragments thereof, single chain antibodies and fragments thereof and miniantibodies, bispecific antibodies, diabodies, triabodies, or di-, oligo- or multimers thereof. Also included are peptidomimetics or peptides derived from the antibodies according to the invention, e.g. they comprise one or several CDR regions, preferably the CDR3 region. Further included are human monoclonal antibodies and peptide sequences which, based on a structure activity connection, are produced through an artificial modeling process (Greer J. et al., J. Med. Chem., 1994, Vol. 37, pp. 1035-1054).

Preferably, the antibody is selected from the group consisting of an intact immunoglobulin (or antibody), a Fv, a scFv (single chain Fv fragment), a Fab, a F(ab')2, an antibody-like domain, an antibody-mimetic domain, a single antibody domain, a multimeric antibody, a peptide or a proteolytic fragment containing the epitope binding region. The term "antibody" in the present invention is used in the most general sense, and encompasses various antibodies and antibody mimetic structures, including, but not limited to, monoclonal antibodies, polyclonal antibodies, multispecific antibodies (e.g., bispecific antibodies), human antibodies, humanized antibodies, deimmunized antibodies, chimeric antibodies, nanobodies, antibody derivatives, antibody fragments, anticalines, DARPins, affibody, affilins, affimers, affitines, alphabody, avimers, fynomers, minibodies and other binding domains, provided that they show desired binding activity for the antigen. An "antibody fragment" refers to a molecule other than an intact antibody that comprises a portion of an intact antibody that binds the antigen to which the intact antibody binds. Examples of antibody fragments include, but are not limited to, Fv, Fab, Fab', Fab'-SH, F(ab')2; diabody; linear antibodies; single-chain antibody molecules (e.g. scFv); and multispecific antibodies consisting of antibody fragments. Fv of VH and VL are also called "nanobodies". The term "mimetic antibody" refers to those organic compounds or binding domains that are not antibody derivatives but that can specifically bind to an antigen, in the same way of the antibodies. They include anticalines, DARPins, affibody, affilins, affimers, affitines, alphabody, avimers, fynomers, minibodies, and others. The term "chimeric" antibody refers to an antibody in which a portion of the heavy and/or light chain is derived from one specific source or species, while the remainder of the heavy and/or light chain is derived from a different source or species.

In the context of the present disclosure, the antibody of the present invention includes modifications of the antibody according to the present invention able to maintain the specificity mentioned above. These changes include, for example, the conjugation to effector molecules such as chemotherapeutic or cytotoxic agents, and/or detectable reporter portions.

The term antibodies may also comprise agents which have been obtained by analysis of data relating to structure-activity relationships. These compounds may also be used as peptidomimetics (Grassy G. et al., Nature Biotechnol., 1998, Vol. 16, pp. 748-752; Greer J. et al., J. Med. Chem., 1994, Vol. 37, pp. 1035-1054).

The term antibody may also include proteins produced by expression of an altered, immunoglobulin-encoding region in a host cell, e.g. "technically modified antibodies" such as synthetic antibodies, chimeric or humanized antibodies, or mixtures thereof, or antibody fragments which partially or completely lack the constant region, e.g. Fv, Fab, Fab' or F(ab)'2 etc. In these technically modified antibodies, e.g., a part or parts of the light and/or heavy chain may be substituted. Such molecules may, e.g., comprise antibodies consisting of a humanized heavy chain and an unmodified light chain (or chimeric light chain), or vice versa. The terms Fv, Fc, Fd, Fab, Fab' or F(ab)2 are used as described in the prior art (Harlow E. and Lane D., in "Antibodies, A Laboratory Manual", Cold Spring Harbor Laboratory, 1988).

The present disclosure also comprises the use of Fab fragments or F(ab)2 fragments which are derived from monoclonal antibodies (mAb), which are directed against CHI3L1. Preferably, the heterologous framework regions and constant regions are selected from the human immunoglobulin classes and isotypes, such as IgG (subtypes 1 to 4), IgM, IgA and IgE. In the course of the immune response, a class switch of the immunoglobulins may occur, e.g. a switch from IgM to IgG; therein, the constant regions are exchanged. A class switch may also be caused in a directed manner by means of genetic engineering methods ("directed class switch recombination"), as is known from the prior art (Esser C. and Radbruch A., Annu. Rev. Immunol., 1990, Vol. 8, pp. 717-735). However, the antibodies according to the present invention need not comprise exclusively human sequences of the immunoglobulin proteins.

The antibodies of the present disclosure also include those for which binding characteristics have been improved by direct mutations, affinity maturation methods, phage display. The affinity or specificity can be modified or improved by mutations in any of the antibody CDRs of the present disclosure.

The antibody-like domain comprises binding proteins structurally related to antibodies, such as T cell receptors. The antibodies of the present disclosure also include functional equivalents that include polypeptides with amino acid sequences substantially identical to the amino acid sequence of the variable or hypervariable regions of the antibodies of the present invention. The antibody of the invention may e.g. have a dissociation constant (KD) of <100 nM, <10 nM, <1 nM, <0.1 nM, <0.01 nM, or <0.001 nM or less, e.g. from 10 -8 M to 10 -13 M, e.g., from 10 -9 M to 10 -13 M. Recombinant and/or biotechnological derivatives as well as fragments of the antibodies described above are included within the invention, provided that the binding activity of the antibodies and their functional specificity is maintained.

In the context of the present invention, the "cancer" or "tumour" includes primary and metastatic tumours, as well as refractory tumours, solid or non-solid tumours. A further aspect of the present disclosure is a nucleic acid encoding the antibody as defined above or hybridizing with the above nucleic acid, or consisting of a correspondent degenerated sequence.

It is disclosed an expression vector encoding the antibody as defined above.

It is within the scope of the disclosure a host cell comprising the nucleic acid as defined above, or the vector as defined above.

The terms "host cell", "host cell line", and "host cell culture" are used interchangeably and refer to cells into which an exogenous nucleic acid has been introduced, including the progeny of such cells. The host cells include "transformants" and "transformed cells," which include the transformed primary cell and the progeny derived therefrom, without taking into account the number of steps. The progeny may be not completely identical in nucleic acid content to a parent cell, but may contain mutations. In the present disclosure mutant progenies are included, which have the same function or biological activity as that for which they have been screened or selected in the originally transformed cell. The nucleic acids of the invention can be used to transform a suitable mammalian host cell. Mammalian cells available as expression hosts are well known and include, for example, CHO and BHK cells. Prokaryotic hosts include, for example, E. coli, Pseudomonas, Bacillus, etc. Antibodies of the disclosure can be fused to additional amino acid residues, such as tags that facilitate their isolation. The term "vector", as used in the present disclosure refers to a nucleic acid molecule capable of propagating another nucleic acid to which it is linked. The term includes the vector as a self-replicating nucleic acid structure as well as the vector incorporated into the genome of a host cell in which it was introduced. Certain vectors are capable of directing the expression of nucleic acids to which they are operably linked. In the present such vectors are referred to as "expression vectors." Any suitable expression vector can be used, for example prokaryotic cloning vectors such as plasmids from E. coli, such as colE1, pCR1, pBR322, pMB9, pUC. Expression vectors suitable for expression in mammalian cells include derivatives of SV-40, adenovirus, retrovirus-derived DNA sequences. The expression vectors useful in the present invention contain at least one expression control sequence that is operatively linked to the sequence or fragment of DNA that must be expressed. The disclosure also relates to a pharmaceutical composition comprising at least the antibody or a synthetic or recombinant fragment thereof as defined above and pharmaceutical acceptable excipients, preferably said composition being for use by parenteral administration, in particular intravenously. The composition comprises an effective amount of the antibody and/or recombinant or synthetic antigen binding fragments thereof. The pharmaceutical compositions are conventional in this field and can be produced by the skilled in the art just based on the common general knowledge. The formulations useful in therapy as described herein may e.g. comprise the antibody as described above, in a concentration from about 0.1 mg/ml to about 100 mg/ml, preferably from 0.1 to 10 mg/ml, more preferably from 0.1 to 5 mg/ml. In other formulations, the antibody concentration may be lower, e.g. at least 100 pg/ml. The antibody of the invention is administered to the patient in one or more treatments. Depending on the type and severity of the disease, a dosage of e.g. about 1 mg/kg to 20 mg/kg of the antibody may be administered, for example in one or more administrations, or by continuous infusion. The antibodies may be administered in combination with other therapeutic agents, in particular with antibodies able to neutralize other receptors involved in tumour growth or angiogenesis. Any method of administration may be used to administer the antibody of the present invention, in particular, for example, the administration may be oral, intravenous, intraperitoneal, subcutaneous, or intramuscular. The antibody may also be administered as a conjugate, which binds specifically to the receptor and releases toxic substances. In particular embodiments, the pharmaceutical composition can be administered in the form of single dosage (for example, tablet, capsule, bolus, etc..). For pharmaceutical applications, the composition may be in the form of a solution, for example, of an injectable solution, emulsion, suspension, or the like. The vehicle can be any vehicle suitable from the pharmaceutical point of view. Preferably, the vehicle used is capable of increasing the entry effectiveness of the molecules into the target cell. In the pharmaceutical composition according to the invention, the inhibitor or suppressor may be associated with other therapeutic agents, such as antagonists of other growth factor receptors involved in tumorigenesis or angiogenesis, such as VEGFR-2, EGFR, PDGFR, receptor kinase inhibitors, BRAF inhibitors, MEK inhibitors, immunomodulatory antibodies, anticancer agents, such as: bevacizumab, ramucirumab, aflibercept, sunitinib, pazopanib, sorafenib, cabozantinib, axitinib, regorafenib, nintedanib, lenvatinib, vemurafenib, dabrafenib, trametinib, chemotherapeutic agents such as methylating agents (temozolomide, dacarbazine), platinum compounds (cisplatin, carboplatin, oxaliplatin), taxanes (paclitaxel, nab-paclitaxel, docetaxel), fluoropyrimidines (5-fluorouracil, capecitabine), topoisomerase I inhibitors (irinotecan, topotecan), poly(ADP-ribose) polymerase inhibitors (PARP) (e.g., olaparib), etc. The pharmaceutical composition is chosen according to the demands of treatment. These pharmaceutical compositions according to the invention may be administered in the form of tablets, capsules, oral preparations, powders, granules, pills, liquid solutions for injection or infusion, suspensions, suppositories, preparations for inhalation. A reference for the formulations is the book by Remington ("Remington: The Science and Practice of Pharmacy", Lippincott Williams & Wilkins, 2000). The skilled in the art will choose the form of administration and the effective dosages, by selecting suitable diluents, adjuvants and/or excipients.

The term "pharmaceutical composition" refers to a preparation that is in such a form as to permit to the biological activity of an active ingredient contained therein to be effective, and which contains no additional components which are unacceptably toxic to a subject to which the formulation may be administered. It is a further aspect of the invention a method for producing the antibody or a synthetic or recombinant fragment thereof as defined above, comprising the steps of culturing the host cell and purifying the antibody or a synthetic or recombinant fragment thereof from the cell culture.

In the context of the present disclosure the term "comprising" also includes the terms "having essentially" or "consisting essentially".

In the present disclosure, the herein mentioned "protein(s)" also comprises the protein encoded by the corresponding orthologous or homologous genes, functional mutants, functional derivatives, functional fragments or analogues, isoform, splice variants thereof.

In the present disclosure "functional" is intended for example as "maintaining their activity".

As used herein "fragments" refers to polypeptides having preferably a length of at least 10 amino acids, more preferably at least 15, at least 17 amino acids or at least 20 amino acids, even more preferably at least 25 amino acids or at least 37 or 40 amino acids, and more preferably of at least 50, or 100, or 150 or 200 or 250 or 300 or 350 or 400 or 450 or 500 amino acids.

In the context of the present disclosure the tumor may be breast cancer, ovarian cancer, small cell lung cancer, non-small cell lung cancer, colorectal cancer, malignant peripheral nerve sheath tumors, cervical cancer, leukemia, prostate, Kaposi's sarcoma, metastatic melanoma, pancreatic cancer, head and neck tumors, meningiomas, basal cell carcinoma, and gliomas.

Optimal pharmaceutical compositions can be determined by one skilled in the art depending upon, for example, the intended route of administration, delivery format and desired dosage. See, for example, REMINGTON'S PHARMACEUTICAL SCIENCES, Id. Such compositions may influence the physical state, stability, rate of in vivo release and rate of in vivo clearance of the antibodies of the invention. Administration routes for the pharmaceutical compositions of the invention include orally, through injection by intravenous, intraperitoneal, intramuscular, intravascular, intraarterial, intraportal, or intralesional routes; by sustained release systems or by implantation devices. The pharmaceutical compositions may be administered by bolus injection or continuously by infusion, or by implantation device. The pharmaceutical composition also can be administered locally via implantation of a membrane, sponge or another appropriate material onto which the desired molecule has been absorbed or encapsulated. Where an implantation device is used, the device may be implanted into any suitable tissue or organ, and delivery of the desired molecule may be via diffusion, timed- release bolus, or continuous administration.

Preferably the molecules of the present disclosure, are for use in combination with a further therapeutic intervention, preferably the further therapeutic intervention is radiotherapy, chemotherapy or a therapeutic agent.

The chemotherapy may be any known chemotherapy used in the art.

The therapeutic agent is an agent commonly used in combination with cancer treatment such as an anti-inflammatory or anti-emetic agent. The therapeutic agent is known in the art.

The present disclosure also relates to the use of an engineered human tumor cell, e.g. CAR T and CAR NK cells, wherein the expression of CHI3L1 was inhibited for inducing or modulating an immune response in respect of a tumor or tumor antigen, and in turn for prophylactic and therapeutic antitumor/anticancer applications. In a further aspect, the present disclosure relates to a composition (e.g. a pharmaceutical or vaccine or immunogenic composition) comprising the above-mentioned engineered human tumor cell as stimulatory molecule or variant thereof and a pharmaceutically acceptable carrier or excipient.

Said suppressor or inhibitor may be used in Adoptive cell transfer (ACT), cell therapy treatment. Unless defined otherwise, the scientific and technological terms and nomenclature used herein have the same meaning as commonly understood by a person of ordinary skill to which this invention pertains. Generally, the procedures for cell cultures, transfection, molecular biology methods and the like, antibody purification and the like, are common methods used in the art. Such standard techniques can be found in reference manuals such as for example Sambrook et al. (1989, Molecular Cloning-A Laboratory Manual, Cold Spring Harbor Laboratories), Ausubel et al. (1994, Current Protocols in Molecular Biology, Wiley, New York), Campbell 1984, in "Monoclonal Antibody Technology: Laboratory Techniques in Biochemistry and Molecular Biology", Elsevier Science Publisher, Amsterdam, The Netherlands), in Harlow et al. 1988 (in: Antibody-A Laboratory Manual, CSH Laboratories), Klein 1982 (in: Immunology: The Science of Self-Nonself Discrimination, Wiley & Sons, N.Y.), in Immunology Today 10: 254 (1989), and in Kanoff, M. E. 1991 : Immunological studies in humans. In Current protocols in Immunology, Vol. 1. Wiley & Sons, New York, p. 7.1.

In order to provide a clear and consistent understanding of terms used in the present description, a number of definitions are provided herein below.

The term "ligand" is well-known in the art of immunology and other ligands include, for example, antibodies which bind a receptor. For certainty, the term "ligand" as used herein is used in its broad sense to refer to a molecule which can bind specifically to another one.

The terms "antigen" or "antigenic determinant" or ("antigenic fragment") are very well-known in the art. The strength of an antigen is often referred to as the antigenicity or immunogenicity and relates to the property (which is often quantifiable) in eliciting or inducing an immune response. The nucleotide and amino acid sequences of human CHI3L1 are well known and are available in the Genbank database, with the following accession codes

| **Nucleotide** | **Accession and version** | **Protein** |
|---|---|---|
| genomic | AC105940.2 (120162..128109) | None |
| genomic | AJ251847.1 | CAB76472.1 |
| genomic | CH471067.1 | EAW91467.1 |
| | | EAW91468.1 |
| genomic | Y08374.1 | CAA69661.1 |
| genomic | Y08378.1 | None |
| mRNA | AB209459.1 | BAD92696.1 |
| mRNA | AK095458.1 | BAG53058.1 |
| mRNA | AK130142.1 | None |
| mRNA | AK130200.1 | None |
| mRNA | AK225266.1 | None |
| mRNA | AK225584.1 | None |
| mRNA | AK312911.1 | BAG35757.1 |
| mRNA | BCOO8568.1 | AAH08568.1 |
| mRNA | BC034684.1 | None |
| mRNA | BC038354.1 | AAH38354.1 |
| mRNA | BC039132.1 | AAH39132.1 |
| mRNA | BT007223.1 | AAP35887.1 |
| mRNA | BX648308.1 | None |
| mRNA | DA167112.1 | None |
| mRNA | M80927.1 | AAA16074.1 |

In a preferred embodiment the nucleotide and amino acid sequences of human CHI3L1 are: Nucleotide CDS of CHI3L1:

>NM_001276.3:82-1233 Homo sapiens chitinase 3 like 1 (CHI3L1), mRNA
ATGGGTGTGAAGGCGTCTCAAACAGGCTTTGTGGTCCTGGTGCTGCTCCAGTGCTGCTCTGCATACAAAC
TGGTCTGCTACTACACCAGCTGGTCCCAGTACCGGGAAGGCGATGGGAGCTGCTTCCCAGATGCCCTTGA

CCGCTTCCTCTGTACCCACATCATCTACAGCTTTGCCAATATAAGCAACGATCACATCGACACCTGGGAG
TGGAATGATGTGACGCTCTACGGCATGCTCAACACACTCAAGAACAGGAACCCCAACCTGAAGACTCTCT
TGTCTGTCGGAGGATGGAACTTTGGGTCTCAAAGATTTTCCAAGATAGCCTCCAACACCCAGAGTCGCCG
GACTTTCATCAAGTCAGTACCGCCATTTCTGCGCACCCATGGCTTTGATGGGCTGGACCTTGCCTGGCTC
TACCCTGGACGGAGAGACAAACAGCATTTTACCACCCTAATCAAGGAAATGAAGGCCGAATTTATAAAGG
AAGCCCAGCCAGGGAAAAAGCAGCTCCTGCTCAGCGCAGCACTGTCTGCGGGGAAGGTCACCATTGACAG
CAGCTATGACATTGCCAAGATATCCCAACACCTGGATTTCATTAGCATCATGACCTACGATTTTCATGGA
GCCTGGCGTGGGACCACAGGCCATCACAGTCCCCTGTTCCGAGGTCAGGAGGATGCAAGTCCTGACAGAT
TCAGCAACACTGACTATGCTGTGGGGTACATGTTGAGGCTGGGGGCTCCTGCCAGTAAGCTGGTGATGGG
CATCCCCACCTTCGGGAGGAGCTTCACTCTGGCTTCTTCTGAGACTGGTGTTGGAGCCCCAATCTCAGGA
CCGGGAATTCCAGGCCGGTTCACCAAGGAGGCAGGGACCCTTGCCTACTATGAGATCTGTGACTTCCTCC
GCGGAGCCACAGTCCATAGAATCCTCGGCCAGCAGGTCCCCTATGCCACCAAGGGCAACCAGTGGGTAGG
ATACGACGACCAGGAAAGCGTCAAAAGCAAGGTGCAGTACCTGAAGGACAGGCAGCTGGCGGGCGCCATG
GTATGGGCCCTGGACCTGGATGACTTCCAGGGCTCCTTCTGCGGCCAGGATCTGCGCTTCCCTCTCACCA
ATGCCATCAAGGATGCACTCGCTGCAACGTAG (SEQ ID NO: 1)

Amino acid sequence of CHI3L1:

MGVKASQTGFVVLVLLQCCSAYKLVCYYTSWSQYREGDGSCFPDALDRFLCTHIIYSFANISND
HIDTWEWNDVTLYGMLNTLKNRNPNLKTLLSVGGWNFGSQRFSKIASNTQSRRTFIKSVPPFLR
THGFDGLDLAWLYPGRRDKQHFTTLIKEMKAEFIKEAQPGKKQLLLSAALSAGKVTIDSSYDIA
KISQHLDFISIMTYDFHGAWRGTTGHHSPLFRGQEDASPDRFSNTDYAVGYMLRLGAPASKLV
MGIPTFGRSFTLASSETGVGAPISGPGIPGRFTKEAGTLAYYEICDFLRGATVHRILGQQVPYATK
GNQWVGYDDQESVKSKVQYLKDRQLAGAMVWALDLDDFQGSFCGQDLRFPLTNAIKDALAA
T (SEQ ID NO: 2)

The nucleotide and amino acid sequences of human IL13ra2 are well known and are available in the Genbank database, with the following accession codes
IL13ra2 accession codes:

| **Nucleotide** | **Accession and version** | **Protein** |
|---|---|---|
| genomic | AH012718.2 | None |
| genomic | AL121878.11 (8039..21708) | None |
| genomic | AY656702.1 | AAT49099.1 |
| genomic | CH471120.2 | EAX02.621.1 |
| | | EAX02622.1 |
| mRNA | AK291957.1 | BA.F84646.1 |
| mRNA | AK308523.1 | None |
| mRNA | AK309444.1 | None |
| mRNA | BC020739.1 | AAH20739.1 |
| mRNA | BC033705.2 | AAH33705.1 |
| mRNA | GQ494004.1 | ACX47590.1 |
| mRNA | U70981.1 | AAB17170.1 |
| mRNA | X95302.1 | CAA.64617.1 |
| mRNA | Y08768.1 | CAA70021.1 |

Also within the context of the disclosure are polypeptides and nucleic acids which are homologous to or substantially identical with, based on sequence, to a polypeptide or nucleic acid of the invention and retain the relevant function.

"Homology" and "homologous" refers to sequence similarity between two peptides or two nucleic acid molecules. Homology can be determined by comparing each position in the aligned sequences. A degree of homology between nucleic acid or between amino acid sequences is a function of the number of identical or matching nucleotides or amino acids at positions shared by the sequences. As the term is used herein, a nucleic acid sequence is "homologous" to another sequence if the two sequences are substantially identical and the functional activity of the sequences is conserved (as used herein, the term 'homologous' does not infer evolutionary relatedness). Two nucleic acid sequences are considered substantially identical if, when optimally aligned (with gaps permitted), they share at least about 50% sequence similarity or identity, or if the sequences share defined functional motifs. In alternative embodiments, sequence similarity in optimally aligned substantially identical sequences may be at least 60%, 70%, 75%, 80%, 85%, 90% or 95%. As used herein, a given percentage of homology between sequences denotes the degree of sequence identity in optimally aligned sequences. An "unrelated" or "non-homologous" sequence shares less than 40% identity, though preferably less than about 25 % identity, with a nucleic acid sequence of the present invention.

Substantially complementary nucleic acids are nucleic acids in which the complement of one molecule is substantially identical to the other molecule. Two nucleic acid or protein sequences are considered "substantially identical" if, when optimally aligned, they share at least about 70% sequence identity. In alternative embodiments, sequence identity may for example be at least 75%, at least 80%, at least 85%, at least 90%, or at least 95%. Optimal alignment of sequences for comparisons of identity may be conducted using a variety of algorithms, such as the local homology algorithm of Smith and Waterman, 1981, Adv. Appl. Math 2. 482, the homology alignment algorithm of Needleman and Wunsch, 1970, J. MoI. Biol. 48:443, the search for similarity method of Pearson and Lipman, 1988, Proc. Natl. Acad. Sci. USA 85: 2444, and the computerised implementations of these algorithms (such as GAP, BESTFIT, FASTA and TFASTA in the Wisconsin Genetics Software Package, Genetics Computer Group, Madison, Wl, U.S.A.). Sequence identity may also be determined using the BLAST algorithm, described in Altschul et al., 1990, J. MoI. Biol. 215:403-10 (using the published default settings). Software for performing BLAST analysis may be available through the National Center for Biotechnology Information . The BLAST algorithm involves first identifying high scoring sequence pairs (HSPs) by identifying short words of length W in the query sequence that either match or satisfy some positive-valued threshold score T when aligned with a word of the same length in a database sequence. T is referred to as the neighbourhood word score threshold. Initial neighbourhood word hits act as seeds for initiating searches to find longer HSPs. The word hits are extended in both directions along each sequence for as far as the cumulative alignment score can be increased. Extension of the word hits in each direction is halted when the following parameters are met: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLAST program may use as defaults a word length (W) of 11 , the BLOSUM62 scoring matrix (Henikoff and Henikoff, 1992, Proc. Natl. Acad. Sci. USA 89: 10915- 10919) alignments (B) of 50, expectation (E) of 10 (or 1 or 0.1 or 0.01 or 0.001 or 0.0001 ), M=5, N=4, and a comparison of both strands. One measure of the statistical similarity between two sequences using the BLAST algorithm is the smallest sum probability (P(N)), which provides an indication of the probability by which a match between two nucleotide or amino acid sequences would occur by chance. In alternative embodiments of the invention, nucleotide or amino acid sequences are considered substantially identical if the smallest sum probability in a comparison of the test sequences is less than about 1, preferably less than about 0.1, more preferably less than about 0.01, and most preferably less than about 0.001.

An alternative indication that two nucleic acid sequences are substantially complementary is that the two sequences hybridize to each other under moderately stringent, or preferably stringent, conditions. Hybridization to filter-bound sequences under moderately stringent conditions may, for example, be performed in 0.5 M NaHP04, 7% sodium dodecyl sulfate (SDS), 1 mM EDTA at 65°C, and washing in 0.2 x SSC/0. 1% SDS at 42°C (see Ausubel, et al. (eds), 1989, Current Protocols in Molecular Biology, Vol. 1 , Green Publishing Associates, Inc., and John Wiley & Sons, Inc., New York, at p. 2.10.3). Alternatively, hybridization to filter-bound sequences under stringent conditions may, for example, be performed in 0.5 M NaHPO4, 7% SDS, 1 mM EDTA at 65<0>C, and washing in 0.1 x SSC/0.1 % SDS at 68°C (see Ausubel, et al. (eds), 1989, supra). Hybridization conditions may be modified in accordance with known methods depending on the sequence of interest (see Tijssen, 1993, Laboratory Techniques in Biochemistry and Molecular Biology - Hybridization with Nucleic Acid Probes, Part I, Chapter 2 Overview of principles of hybridization and the strategy of nucleic acid probe assays", Elsevier, New York). Generally, stringent conditions are selected to be about 5[deg.]C lower than the thermal melting point for the specific sequence at a defined ionic strength and pH.

The term "vector" is commonly known in the art and defines e.g., a plasmid DNA, phage DNA, viral DNA and the like, which can serve as a vehicle into which the nucleic acid of the present invention can be cloned. Numerous types of vectors exist and are well known in the art. In a particular embodiment, the vector is a viral vector which can introduce a molecule, e.g. a chimeric co-stimulatory molecule, in a cell or in a living organism. In an embodiment the viral vector is a retroviral vector, a lentiviral vector, an adenoviral vector or an adeno-associated vector (AAV). Various genes and nucleic acid sequences of the invention may be recombinant sequences. The term "recombinant" means that something has been recombined, so that when made in reference to a nucleic acid construct the term refers to a molecule that is comprised of nucleic acid sequences that are joined together or produced by means of molecular biological techniques. The term "recombinant" when made in reference to a protein or a polypeptide refers to a protein or polypeptide molecule which is expressed using a recombinant nucleic acid construct created by means of molecular biological techniques. The term "recombinant" when made in reference to genetic composition refers to a gamete or progeny or cell or genome with new combinations of alleles that did not occur in the parental genomes. Recombinant nucleic acid constructs may include a nucleotide sequence which is ligated to, or is manipulated to become ligated to, a nucleic acid sequence to which it is not ligated in nature, or to which it is ligated at a different location in nature. Referring to a nucleic acid construct as "recombinant" therefore indicates that the nucleic acid molecule has been manipulated using genetic engineering, i.e. by human intervention. Recombinant nucleic acid constructs may for example be introduced into a host cell by transformation. Such recombinant nucleic acid constructs may include sequences derived from the same host cell species or from different host cell species, which have been isolated and reintroduced into cells of the host species. Recombinant nucleic acid construct sequences may become integrated into a host cell genome, either as a result of the original transformation of the host cells, or as the result of subsequent recombination and/or repair events.

The term "expression" defines the process by which a gene is transcribed into mRNA (transcription), the mRNA is then being translated (translation) into one polypeptide (or protein) or more.

The terminology "expression vector" defines a vector or vehicle as described above but designed to enable the expression of an inserted sequence following transformation or transfection into a host. The cloned gene (inserted sequence) is usually placed under the control of control element or transcriptionally regulatory sequences such as promoter sequences. The placing of a cloned gene under such control sequences is often referred to as being operably linked to control elements or sequences.

A first nucleic acid sequence is "operably-linked" with a second nucleic acid sequence when the first nucleic acid sequence is placed in a functional relationship with the second nucleic acid sequence. For instance, a promoter is operably-linked to a coding sequence if the promoter affects the transcription or expression of the coding sequences. Generally, operably-linked DNA sequences are contiguous and, where necessary to join two protein coding regions, in reading frame. However, since for example enhancers generally function when separated from the promoters by several kilobases and intronic sequences may be of variable lengths, some polynucleotide elements may be operably-linked but not contiguous. "Transcriptional regulatory element" is a generic term that refers to DNA sequences, such as initiation and termination signals, enhancers, and promoters, splicing signals, polyadenylation signals which induce or control transcription of protein coding sequences with which they are operably-linked.

Operably-linked sequences may also include two segments that are transcribed onto the same RNA transcript. Thus, two sequences, such as a promoter and reporter sequence are operably linked if transcription commencing in the promoter will produce an RNA transcript of the reporter sequence. In order to be "operably-linked" it is not necessary that two sequences be immediately adjacent to one another.

Expression control sequences will vary depending on whether the vector is designed to express the operably-linked gene in a prokaryotic or eukaryotic host or both (shuttle vectors) and can additionally contain transcriptional elements such as enhancer elements, termination sequences, tissue-specificity elements, and/or translational initiation and termination sites.

Prokaryotic expressions are useful for the preparation of large quantities of the protein encoded by the DNA sequence of interest. This protein can be purified according to standard protocols that take advantage of the intrinsic properties thereof, such as size and charge (e. g. SDS gel electrophoresis, gel filtration, centrifugation, ion exchange chromatography, etc.). In addition, the protein of interest can be purified via affinity chromatography using polyclonal or monoclonal antibodies or a specific ligand. The purified protein can be used for therapeutic applications. Prokaryotically expressed eukaryotic proteins are often not glycosylated.

The DNA (or RNA) construct can be a vector comprising a promoter that is operably linked to an oligonucleotide sequence of the present invention, which is in turn, operably linked to a heterologous gene, such as the gene for the luciferase reporter molecule. "Promoter" refers to a DNA regulatory region capable of binding directly or indirectly to RNA polymerase in a cell and initiating transcription of a downstream (3' direction) coding sequence. For purposes of the present invention, the promoter is preferably bound at its 3' terminus by the transcription initiation site and extends upstream (5' direction) to include the minimum number of bases or elements necessary to initiate transcription at levels detectable above background. Within the promoter will be found a transcription initiation site (conveniently defined by mapping with S1 nuclease), as well as protein binding domains (consensus sequences) responsible for the binding of RNA polymerase. Eukaryotic promoters will often, but not always, contain "TATA" boxes and "CCAT" boxes. Prokaryotic promoters contain -10 and-35 consensus sequences, which serve to initiate transcription and the transcript products contain Shine-Dalgarno sequences, which serve as ribosome binding sequences during translation initiation. Non-limiting examples of vectors which can be used in accordance with the present invention include adenoviral vectors, poxviral vectors, VSV-derived vectors and retroviral vectors. Such vectors and others are well-known in the art. As used herein, the designation "functional derivative" or "functional variant" denotes, in the context of a functional derivative of a sequence whether a nucleic acid or amino acid sequence, a molecule that retains a biological activity (either function or structural) that is substantially similar to that of the original sequence. This functional derivative or equivalent may be a natural derivative or may be prepared synthetically. Such derivatives include amino acid sequences having substitutions, deletions, or additions of one or more amino acids, provided that the biological activity of the protein is conserved. The same applies to derivatives of nucleic acid sequences which can have substitutions, deletions, or additions of one or more nucleotides, provided that the biological activity of the sequence is generally maintained. When relating to a protein sequence, the substituting amino acid generally has chemico-physical properties which are similar to that of the substituted amino acid. The similar chemico-physical properties include, similarities in charge, bulkiness, hydrophobicity, hydrophylicity and the like. The term "functional derivatives" is intended to include "fragments", "segments", "variants", "analogs" or "chemical derivatives" of the subject matter of the present invention. In an embodiment, the above-mentioned derivative, variant or fragment is an "antigenic derivative, variant or fragment" (e.g., which has the capacity to induce/elicit an immune response against the parental antigen).

Thus, the term "variant" refers herein to a protein or nucleic acid molecule which is substantially similar in structure and biological activity to the protein or nucleic acid of the present invention but is not limited to a variant which retains all of the biological activities of the parental protein, for example.

The functional derivatives of the present invention can be synthesized chemically or produced through recombinant DNA technology. All these methods are well known in the art.

As used herein, "chemical derivatives" is meant to cover additional chemical moieties not normally part of the subject matter of the invention. Such moieties could affect the physicochemical characteristic of the derivative (e.g. solubility, absorption, half-life, decrease of toxicity and the like). Such moieties are exemplified in Remington's Pharmaceutical Sciences (1980). Methods of coupling these chemical-physical moieties to a polypeptide or nucleic acid sequence are well known in the art.

As used herein, the terms "molecule", "compound", or "agent" are used interchangeably and broadly to refer to natural, synthetic or semi-synthetic molecules or compounds. The term "molecule" therefore denotes for example chemicals, macromolecules, cell or tissue extracts (from plants or animals) and the like. Non-limiting examples of molecules include nucleic acid molecules, peptides, antibodies, carbohydrates and pharmaceutical agents. The agents can be selected and screened by a variety of means including random screening, rational selection and by rational design using for example protein or ligand modeling methods such as computer modeling. The terms "rationally selected" or "rationally designed" are meant to define compounds which have been chosen based on the configuration for example of interacting domains of the present invention. As will be understood by the person of ordinary skill, macromolecules having non-naturally occurring modifications are also within the scope of the term "molecule". For example, peptidomimetics, well known in the pharmaceutical industry and generally referred to as peptide analogs can be generated by modeling as mentioned above. Similarly, the polypeptides described herein can be modified to enhance their stability. It should be understood that in most cases this modification should not alter the biological activity of the interaction domain. The molecules identified in accordance with the teachings of the present disclosure have a therapeutic value in diseases or conditions in which the antibody-dependent cell-mediated cytotoxicity (ADCC) dependent therapies are not efficient. As defined above, the term "ligand" also encompasses molecules such as peptides, antibodies and carbohydrates.

For certainty, the sequences and polypeptides useful to practice the disclosure include without being limited thereto mutants, homologs, subtypes, alleles and the like. It will be clear to the person of ordinary skill that whether an interaction domain of the present disclosure, variant, derivative, or fragment thereof retains its function in binding to its partner can be readily determined by using the teachings and assays of the present disclosure and the general teachings of the art.

Also within the context of the present disclosure is the in vivo administration of a nucleic acid as disclosed herein to a subject, such as gene therapy methods.

Nucleic acids may be delivered to cells in vivo using methods such as direct injection of DNA, receptor-mediated DNA uptake, viral-mediated transfection or non-viral transfection and lipid based transfection, all of which may involve the use of gene therapy vectors. Direct injection has been used to introduce naked DNA into cells in vivo (see e.g., Acsadi et al. (1991 ) Nature 332:815-818; Wolff et al. (1990) Science 247:1465-1468). A delivery apparatus (e.g., a "gene gun") for injecting DNA into cells in vivo may be used. Such an apparatus may be commercially available (e.g., from BioRad). Naked DNA may also be introduced into cells by complexing the DNA to a cation, such as polylysine, which is coupled to a ligand for a cell-surface receptor (see for example Wu, G. and Wu, C. H. (1988) J. Biol. Chem. 263:14621 ; Wilson et al. (1992) J. Biol. Chem. 267:963-967; and U.S. Pat. No. 5,166,320). Binding of the DNA-ligand complex to the receptor may facilitate uptake of the DNA by receptor-mediated endocytosis. A DNA-ligand complex linked to adenovirus capsids which disrupt endosomes, thereby releasing material into the cytoplasm, may be used to avoid degradation of the complex by intracellular lysosomes (see for example Curiel et al. (1991 ) Proc. Natl. Acad. Sci. USA 88:8850; Cristiano et al. (1993) Proc. Natl. Acad. Sci. USA 90:2122-2126). Defective retroviruses are well characterized for use as gene therapy vectors (for a review see Miller, A. D. (1990) Blood 76:271 ). Protocols for producing recombinant retroviruses and for infecting cells in vitro or in vivo with such viruses can be found in Current Protocols in Molecular Biology, Ausubel, F. M. et al. (eds.) Greene Publishing Associates, (1989), Sections 9.10-9.14 and other standard laboratory manuals. Examples of suitable retroviruses include pLJ, pZIP, pWE and pEM which are well known to those skilled in the art. Examples of suitable packaging virus lines include .psi.Crip, .psi.Cre, .psi.2 and .psi.Am. Retroviruses have been used to introduce a variety of genes into many different cell types, including epithelial cells, endothelial cells, lymphocytes, myoblasts, hepatocytes, bone marrow cells, in vitro and/or in vivo (see for example Eglitis, et al. (1985) Science 230:1395-1398; Danos and Mulligan (1988) Proc. Natl. Acad. Sci. USA 85:6460-6464; Wilson et al. (1988) Proc. Natl. Acad. Sci. USA 85:3014-3018; Armentano et al. (1990) Proc. Natl. Acad. Sci. USA 87:6141-6145; Huber et al. (1991) Proc. Natl. Acad. Sci. USA 88:8039-8043; Ferry et al. (1991) Proc. Natl. Acad. Sci. USA 88:8377-8381 ; Chowdhury et al. (1991 ) Science 254:1802-1805; van Beusechem et al. (1992) Proc. Natl. Acad. Sci. USA 89:7640-7644; Kay et al. (1992) Human Gene Therapy 3:641-647; Dai et al. (1992) Proc. Natl. Acad. Sci. USA 89:10892-10895; Hwu et al. (1993) J. Immunol. 150:4104-4115; U.S. Pat. No. 4,868,116; U.S. Pat. No. 4,980,286; PCT Application WO 89/07136; PCT Application WO 89/02468; PCT Application WO 89/05345; and PCT Application WO 92/07573).

Adeno-associated virus (AAV) may be used as a gene therapy vector for delivery of DNA for gene therapy purposes. AAV is a naturally occurring defective virus that requires another virus, such as an adenovirus or a herpes virus, as a helper virus for efficient replication and a productive life cycle (Muzyczka et al. Curr. Topics in Micro, and Immunol. (1992) 158:97-129). AAV may be used to integrate DNA into non-dividing cells (see for example Flotte et al. (1992) Am. J. Respir. Cell. MoI. Biol. 7:349-356; Samulski et al. (1989) J. Virol. 63:3822-3828; and McLaughlin et al. (1989) J. Virol. 62:1963-1973). An AAV vector such as that described in Tratschin et al. (1985) MoI. Cell. Biol. 5:3251-3260 may be used to introduce DNA into cells (see for example Hermonat et al. (1984) Proc. Natl. Acad. Sci. USA 81:6466-6470; Tratschin et al. (1985) MoI. Cell. Biol. 4:2072-2081 ; Wondisford et al. (1988) MoI. Endocrinol. 2:32-39; Tratschin et al. (1984) J. Virol. 51 :611-619; and Flotte et al. (1993) J. Biol. Chem. 268:3781-3790). Lentiviral gene therapy vectors may also be adapted for use according to the disclosure.

From the specification and appended claims, the term therapeutic agent should be taken in a broad sense so as to also include a combination of at least two such therapeutic agents. Further, the DNA segments or proteins according to the present-invention can be introduced into individuals in a number of ways. E.g. cells can be isolated from an individual afflicted or at risk of suffering from a cancer, transfected with a DNA construct according to the invention and reintroduced to the afflicted individual in a number of ways, including intravenous injection. Alternatively, the DNA construct can be administered directly to the afflicted individual, for example, by injection in the thymus. The DNA construct can also be delivered through a vehicle such as a liposome, which can be designed to be targeted to a specific cell type, and engineered to be administered through different routes. Of course, proteins or peptides can also be administered. A person of ordinary skill can adapt the transfection method, type of cells transfected, type of disease or condition, co- stimulus (general or specific) etc to meet particular needs.

Compositions described herein should contain the active agent (e.g. fusion protein, peptide, nucleic acid, and molecule, or antigen, or antibody, or APC) in an amount effective to achieve the desired ADCC activation while avoiding adverse side effects. Typically, the nucleic acids in accordance with the present invention can be administered to mammals (e.g. humans) in doses ranging from 0.005 to 1 mg per kg of body weight per day of the mammal which is treated. Pharmaceutically acceptable preparations and salts of the active agent are within the scope of the present disclosure and are well known in the art (Remington's Pharmaceutical Science, 16th ed., Mack ed.). The disclosure therefore further provides a composition comprising an active agent and a pharmaceutically acceptable carrier. For the administration of polypeptides, antagonists, agonists and the like, the amount administered should be chosen so as to avoid adverse side effects. The dosage will be adapted by the clinician in accordance with conventional factors such as the extent of the disease and different parameters from the patient. Typically, 0.001 to 50 mg/kg/day will be administered to the mammal.

As used herein, the compositions are administered essentially intra-tumorally or in ex vivo tumor cell cultures, it may by conventional routes known the field, in such as to a mucosal (e.g., ocular, intranasal, pulmonary, oral, gastric, intestinal, rectal, vaginal, or urinary tract) surface, via a parenteral (e.g., subcutaneous, intradermal, intramuscular, intravenous, or intraperitoneal) route, or topical administration (e.g. via a patch). The choice of administration route depends upon a number of parameters, such as the adjuvant associated with the polypeptide.

If a mucosal adjuvant is used, the intranasal or oral route is preferred. If a lipid formulation or an aluminum compound is used, the parenteral route is preferred with the subcutaneous or intramuscular route being most preferred. For use in a composition of the invention, a polypeptide or derivative thereof may be formulated into or with liposomes, preferably neutral or anionic liposomes, microspheres, ISCOMS, or virus-like-particles (VLPs) to facilitate delivery and/or enhance the immune response. These compounds are readily available to one skilled in the art; for example, see Liposomes: A Practical Approach, RCP New Ed, IRL press (1990).

Adjuvants other than liposomes and the like are also used and are known in the art.

A polynucleotide can also be useful as a therapy. There are two major routes, either using a viral or bacterial host as gene delivery vehicle (vector) or administering the gene in a free form, e.g., inserted into a plasmid. Therapeutic or prophylactic efficacy of a polynucleotide of the disclosure is evaluated as described below.

Accordingly, a further aspect of the disclosure provides (iv) a cell (e.g. a human tumor cell) transfected or transformed with the nucleic acid or vector herein disclosed. Treatment may be effected in a single dose or repeated at intervals. The appropriate dosage depends on various parameters understood by skilled artisans such as the route of administration or the condition of the mammal to be treated (weight, age and the like). Vectors available in the art include viral vectors such as adenoviruses and poxviruses as well as bacterial vectors, e.g., Shigella, Salmonella, Vibrio cholerae, Lactobacillus, Bacille Calmette-Guerin (BCG), and Streptococcus.

An example of an adenovirus vector, as well as a method for constructing an adenovirus vector capable of expressing a nucleic acid molecule of the invention, are described in U.S. Patent No. 4,920,209. Poxvirus vectors include vaccinia and canary poxvirus, described in U.S. Patent No. 4,722,848 and U.S. Patent No. 5,364,773, respectively (also see, e.g., Tartaglia et al., Virology (1992) 188:217) for a description of a vaccinia virus vector and Taylor et al., Vaccine (1995) 13:539 for a description of a canary pox vector). Poxvirus vectors capable of expressing a polynucleotide of the invention are obtained by homologous recombination as described in Kieny et al., Nature (1984) 312:163 so that the polynucleotide of the invention is inserted in the viral genome under appropriate conditions for expression in mammalian cells. Generally, the dose of viral vector, for therapeutic or prophylactic use, can be of from about 1×10⁴ to about 1×10¹¹, advantageously from about 1×10⁷ to about 1×10¹⁰, preferably of from about 1×10⁷ to about 1×10⁹ plaque-forming units per kilogram. Preferably, viral vectors are administered parenterally; for example, in 3 doses, 4 weeks apart. It is preferable to avoid adding a chemical adjuvant to a composition containing a viral vector of the invention and thereby minimizing the immune response to the viral vector itself.

The composition of the present disclosure may further contain an adjuvant. A number of adjuvants are known to those skilled in the art. Examples of adjuvants are described below. Use of the nucleic acids of the invention include their administration to a mammal as a vaccine or an immunogenic agent, for therapeutic or prophylactic purposes. Such nucleic acids are used in the form of DNA as part of a plasmid that is unable to replicate in a mammalian cell and unable to integrate into the mammalian genome. Typically, such a DNA molecule is placed under the control of a promoter suitable for expression in a mammalian cell. The promoter functions either ubiquitously or tissue-specifically. Examples of non-tissue specific promoters include the early Cytomegalovirus (CMV) promoter (described in U.S. Patent No. 4,168,062) and the Rous Sarcoma Virus promoter (described in Norton & Coffin, MoI. Cell Biol. (1985) 5:281). An example of a tissue-specific promoter is the desmin promoter which drives expression in muscle cells (Li et al., Gene (1989) 78:243, Li & Paulin, J. Biol. Chem. (1991 ) 266:6562 and Li & Paulin, J. Biol. Chem. (1993) 268:10403). Use of promoters is well-known to those skilled in the art. Useful vectors are described in numerous publications, specifically WO 94/21797 and Hartikka et al., Human Gene Therapy (1996) 7:1205. Polynucleotides which are used as vaccines encode either a precursor or a mature form of the corresponding polypeptide. In the precursor form, the signal peptide is either homologous or heterologous. In the latter case, a eukaryotic leader sequence such as the leader sequence of the tissue-type plasminogen factor (tPA) is preferred. Standard techniques of molecular biology for preparing and purifying polynucleotides are used in the preparation of polynucleotide therapeutics . For use as a vaccine, a polynucleotide of the invention is formulated according to various methods outlined below. One method utilizes the polynucleotide in a naked form, free of any delivery vehicles. Such a polynucleotide is simply diluted in a physiologically acceptable solution such as sterile saline or sterile buffered saline, with or without a carrier. When present, the carrier preferably is isotonic, hypotonic, or weakly hypertonic, and has a relatively low ionic strength, such as provided by a sucrose solution, e.g., a solution containing 20% sucrose.

An alternative method utilizes the polynucleotide in association with agents that assist in cellular uptake. Examples of such agents are (i) chemicals that modify cellular permeability, such as bupivacaine (see, e.g., WO 94/16737), (ii) liposomes for encapsulation of the polynucleotide, or (iii) cationic lipids or silica, gold, or tungsten microparticles which associate themselves with the polynucleotides.

Anionic and neutral liposomes are well-known in the art (see, e.g., Liposomes: A Practical Approach, RPC New Ed, IRL press (1990), for a detailed description of methods for making liposomes) and are useful for delivering a large range of products, including polynucleotides. Cationic lipids are also known in the art and are commonly used for gene delivery. Such lipids include Lipofectin(TM) also known as DOTMA (N-[1-(2,3- dioleyloxy)propyl]-N,N,N-trimethylammonium chloride), DOTAP (1 ,2-bis(oleyloxy)- 3-(trimethylammonio)propane), DDAB (dimethyldioctadecylammonium bromide), DOGS (dioctadecylamidologlycyl spermine) and cholesterol derivatives such as DC-Choi (3 beta-(N-(N',N'-dimethyl aminomethane)-carbamoyl) cholesterol). A description of these cationic lipids can be found in EP 187,702, WO 90/11092, U.S. Patent No. 5,283,185, WO 91/15501 , WO 95/26356, and U.S. Patent No. 5,527,928. Cationic lipids for gene delivery are preferably used in association with a neutral lipid such as DOPE (dioleyl phosphatidylethanolamine), as described in WO 90/11092 as an example. Formulations containing cationic liposomes may optionally contain other transfection-facilitating compounds. A number of them are described in WO 93/18759, WO 93/19768, WO 94/25608, and WO 95/02397. They include spermine derivatives useful for facilitating the transport of DNA through the nuclear membrane (see, for example, WO 93/18759) and membrane-permeabilizing compounds such as GALA, Gramicidine S, and cationic bile salts (see, for example, WO 93/19768).

Gold or tungsten microparticles are used for gene delivery, as described in WO 91/00359, WO 93/17706, and Tang et al. Nature (1992) 356:152. The microparticle-coated polynucleotide is injected via intradermal or intraepidermal routes using a needleless injection device ("gene gun"), such as those described in U.S. Patent No. 4,945,050, U.S. Patent No. 5,015,580, and WO 94/24263.

The amount of DNA to be used in a vaccine recipient depends, e.g., on the strength of the promoter used in the DNA construct, the immunogenicity of the expressed gene product, the condition of the mammal intended for administration (e.g., the weight, age, and general health of the mammal), the mode of administration, and the type of formulation. In general, a therapeutically or prophylactically effective dose from about 1 µg to about 1 mg, preferably, from about 10 µg to about 800 [mu]g and, more preferably, from about 25 µg to about 250 µg, can be administered to human adults. The administration can be achieved in a single dose or repeated at intervals.

Although not absolutely required, such a composition can also contain an adjuvant. If so, a systemic adjuvant that does not require concomitant administration in order to exhibit an adjuvant effect is preferable such as, e.g., QS21, which is described in U.S. Patent No. 5,057,546.

Treatment is achieved in a single dose or repeated as necessary at intervals, as can be determined readily by one skilled in the art. For example, a priming dose is followed by three booster doses at weekly or monthly intervals. An appropriate dose depends on various parameters including the recipient (e.g., adult or infant), the particular vaccine antigen, the route and frequency of administration, the presence/absence or type of adjuvant, and the desired effect (e.g., protection and/or treatment), as can be determined by one skilled in the art. In an embodiment, a polypeptide of the invention, administered as a vaccine, is administered by a mucosal route in an amount from about 10 µg to about 500 mg, preferably from about 1 mg to about 200 mg. For the parenteral route of administration, the dose usually does not exceed about 1 mg, preferably about 100 µg. When used as vaccine agents, polypeptides and polynucleotides of the invention may be used sequentially as part of a multistep immunization process. For example, a mammal is initially primed with a vaccine vector of the invention such as an adenovirus, e.g., via the parenteral route, and then boosted twice with the polypeptide encoded by the vaccine vector, e.g., via the mucosal route. In another example, liposomes associated with a polypeptide or derivative of the invention are also used for priming, with boosting being carried out mucosally using a soluble polypeptide or derivative of the invention in combination with a mucosal adjuvant (e.g., LT). Examples of adjuvants useful in any of the vaccine/immunogenic compositions described above are as follows. Adjuvants for parenteral administration include aluminum compounds, such as aluminum hydroxide, aluminum phosphate, and aluminum hydroxy phosphate. The antigen is precipitated with, or adsorbed onto, the aluminum compound according to standard protocols. Other adjuvants, such as RIBI (ImmunoChem, Hamilton, MT), are used in parenteral administration. Adjuvants for mucosal administration include bacterial toxins, e.g., the cholera toxin (CT), the E. coli heat-labile toxin (LT), the Clostridium difficile toxin A and the pertussis toxin (PT), or combinations, subunits, toxoids, or mutants thereof such as a purified preparation of native cholera toxin subunit B (CTB). Fragments, homologs, derivatives, and fusions to any of these toxins are also suitable, provided that they retain adjuvant activity. Preferably, a mutant having reduced toxicity is used. Suitable mutants are described, e.g., in WO 95/17211 (Arg-7-Lys CT mutant), WO 96/06627 (Arg-192-Gly LT mutant), and WO 95/34323 (Arg-9-Lys and Glu-129-Gly PT mutant). Additional LT mutants that are used in the methods and compositions of the invention include, e.g., Ser-63-Lys, Ala-69Gly, Glu-110-Asp, and Glu-112-Asp mutants. Other adjuvants, such as a bacterial monophosphoryl lipid A (MPLA) of, e.g., E. coli, Salmonella minnesota, Salmonella typhimurium, or Shigella flexneri; saponins, or polylactide glycolide (PLGA) microspheres, is also be used in mucosal administration.

Adjuvants useful for both mucosal and parenteral administrations include polyphosphazene (WO 95/02415), DC-chol (3 b'N'N'.N'-dimethyl aminomethane)-carbamoyl) cholesterol; U.S. Patent No. 5,283,185 and WO 96/14831 ) and QS-21 (WO 88/09336).

The composition for cancer treatment can be used in conjunction with existing chemotherapy drugs or be made as a mixture with them. Such a chemotherapy drug include, for example, alkylating agents, nitrosourea agents, antimetabolites, antitumor antibiotics, alkaloids derived from plant, topoisomerase inhibitors, hormone therapy medicines, hormone antagonists, aromatase inhibitors, P-glycoprotein inhibitors, platinum complex derivatives, other immunotherapeutic drugs, and other anticancer agents. Further, they can be used together with hypoleukocytosis (neutrophil) medicines that are cancer treatment adjuvant, thrombopenia medicines, antiemetic drugs, and cancer pain medicines for patient's QOL recovery or be made as a mixture with them. The composition and the other agent(s) (e.g. a chemotherapeutic agent) can be administered concomitantly or sequentially.

The composition for cancer treatment can be used together with immunopotentiative substance(s) or be made as a mixture with them. Such immunopotentiative substances include, for example, various cytokines and a tumor antigen, etc. Cytokines that stimulate immune reactions include, for example, GM-CSF, M-CSF, G-CSF, interferon-[beta] and Y, IL-1 , IL-2, IL-3, and IL-12, anti-CD3 antibodies can also improve the immune reactions. The composition of the present invention and immunopotentiative substance(s) (e.g. a cytokine or a chemokine) can be administered concomitantly or sequentially.

The term "cancer" and "tumor" are exchangeable in the present invention. The term "treating cancer" or "treatment of cancer" as used herein includes at least one of the following features (prophylactic /therapeutic results): alleviation of the symptoms associated with the cancer, a reduction in the extent of the cancer (e.g. a reduction in tumor growth), a stabilization of the state of the cancer (e.g. an inhibition of tumor growth), a prevention of further spread of the cancer (e.g. a metastasis), a prevention of the occurrence or recurrence of a cancer, a delaying or retardation of the progression of the cancer (e.g. a reduction in tumor growth) or an improvement in the state of the cancer (e.g. a reduction in tumor size).

A "therapeutically effective amount" refers to an amount effective, at dosages and for periods of time necessary, to achieve the above-mentioned desired therapeutic result. A therapeutically effective amount may vary according to factors such as the disease state, age, sex, and weight of the individual, and the ability of the compound to elicit a desired response in the individual. Dosage regimens may be adjusted to provide the optimum therapeutic response. A therapeutically effective amount is also one in which any toxic or detrimental effects of the compound are outweighed by the therapeutically beneficial effects. A "prophylactically effective amount" refers to an amount effective, at dosages and for periods of time necessary, to achieve the desired prophylactic result, such as preventing or inhibiting onset or progression of cancer and associated symptoms and disease. A prophylactically effective amount can be determined as described above for the therapeutically effective amount. For any particular subject, specific dosage regimens may be adjusted over time according to the individual need and the professional judgement of the person administering or supervising the administration of the compositions.

The cancers to which the compositions and methods can be applied include swellings and true tumors including benign and malignant tumors. Specific examples of such tumors are gliomas such as astrocytoma, glioblastoma, medulloblastoma, oligodendroglioma, ependymoma and choroid plexus papilloma; cerebral tumors such as meningioma, pituitary adenoma, neurioma, congenital tumor, metastatic cerebral tumor; squamous cell carcinoma, lymphoma, a variety of adenomas and pharyngeal cancers resulted from these adenomas such as epipharyngeal cancer, nasopharyngeal cancer and hypopharyngeal cancer; laryngeal cancer, thymoma; mesothelioma such as pleural mesothelioma, peritoneal mesothelioma and pericardial mesothelioma; breast cancers such as thoracic duct cancer, lobular carcinoma and papillary cancer; lung cancers such as small cell carcinoma, adenocarcinoma, squamous cell carcinoma, large cell carcinoma and adenosquamous carcinoma; gastric carcinoma; esophageal carcinomas such as cervical esophageal carcinomas, thoracic esophageal carcinomas and abdominal esophageal carcinomas; carcinomas of large intestine such as rectal carcinoma, S-like (sigmoidal) colon carcinoma, ascending colon carcinoma, lateral colon carcinoma, cecum carcinoma and descending colon carcinoma; hepatomas such as hepatocellular carcinoma, intrahepatic hepatic duct carcinoma, hepatocellular blastoma and hepatic duct cystadenocarcinoma; pancreatic carcinoma; pancreatic hormone-dependent tumors such as insulinoma, gastrinoma, VIP-producing adenoma, extrahepatic hepatic duct carcinoma, hepatic capsular carcinoma, perial carcinoma, renal pelvic and uretal carcinoma; urethral carcinoma; renal cancers such as renal cell carcinoma (Grawitz tumor), Wilms<1> tumor (nephroblastoma) and renal angiomyolipoma; testicular cancers or germ cell tumors such as seminoma, embryonal carcinoma, vitellicle tumor, choriocarcinoma and teratoma; prostatic cancer, bladder cancer, carcinoma of vulva; hysterocarcinomas such as carcinoma of uterine cervix, uterine corpus cancer and solenoma; hysteromyoma, uterine sarcoma, villous diseases, carcinoma of vagina; ovarian germ cell tumors such as dysgerminoma, vitellicle tumor, premature teratoma, dermoidal cancer and ovarian tumors such as ovarian cancer; melanomas such as nevocyte and melanoma; skin lymphomas such as mycosis fungoides, skin cancers such as endoepidermal cancers resulted from skin cancers, prodrome or the like and spinocellular cancer, soft tissue sarcomas such as fibrous histiocytomatosis, liposarcoma, rhabdomyosarcoma, leiomyosarcoma, synovial sarcoma, sarcoma fibroplasticum (fibrosarcoma), neurioma, hemangiosarcoma, fibrosarcoma, neurofibrosarcoma, perithelioma (hemangiopericytoma) and alveolar soft part sarcoma, lymphomas such as Hodgkin lymphoma and non-Hodgkin lymphoma, myeloma, plasmacytoma, acute myelocytic (myeloid) leukemia and chronic myeloid leukemia, leukemia such as adult T-cell leukemic lymphoma and chronic lymphocytic leukemia, chronic myeloproliferative diseases such as true plethora, essential thrombocythemia and idiopathic myelofibrosis, lymph node enlargement (or swelling), tumor of pleural effusion, ascitic tumor, other various kinds of adenomas, lipoma, fibroma, hemangeoma, myoma, fibromyoma and endothelioma.

In a further aspect, the disclosure provides a recombinant cell comprising the above-mentioned vector. In a further embodiment, the above-mentioned cell is an antigen-presenting cell (e.g. a tumor cell).

In a further aspect, the disclosure provides a composition comprising the above-mentioned cell and a pharmaceutically acceptable carrier or excipient.

Within the scope of the disclosure are cells (e.g. host cells) transfected or transformed with the nucleic acid or the vector of the invention. Methods for transforming/transfecting host cells with nucleic acids/vectors are well-known in the art and depend on the host system selected as described in Ausubel et al. (Ausubel et al., Current Protocols in Molecular Biology, John Wiley & Sons Inc., 1994). The terms "transformation" and "transfection" refer to techniques for introducing foreign nucleic acid into a host cell, including calcium phosphate or calcium chloride co-precipitation, DEAE-dextran-mediated transfection, lipofection, electroporation, microinjection and viral-mediated transfection. Host cells transfected or transformed with the nucleic acid or vector of the invention can be used as a vaccine (e.g., autologous cell vaccine) in order to induce or increase an immune response against an antigenic epitope or antigen (e.g., STEAP) in a subject. For example, host cells (e.g., APCs, dendritic cells) may be removed from a subject (e.g., a cancer patient), transfected or transformed in accordance with the present invention and re-administered to the patient. Of course, known steps for further cultivating or modifying these cells could be carried- out prior to re-injecting/transplanting them into a subject. For example, cytokines/chemokines or mitogens or molecules could be added to the culture medium. the claims, the word "comprising" is used as an open-ended term, substantially equivalent to the phrase "including, but not limited to".

The invention will be illustrated by means of non-limiting examples in reference to the following figures.
**Fig 1****:** ADCC of NK cells from healthy donors is negatively modulated by sera of Trastuzumab resistant patients (PD) but not complete remission patients (RC)
**Fig 2****:** CHI3L1 levels correlate with disease progression and unfavorable prognosis In breast cancer, (a) levels of CHI3L1 in the sera of HER2 breast cancer PO patients (N=10), CR patients (N=10) and healthy controls (N=9) during treatment (Left pane) or at study endpoint (right panel), (b) Overall survival of HER-2 positive breast cancer patients according to CHI3L1 expression in primary tumor (n=95, Cutoff--Median), source: Kmplotter (breast cancer), (c) overall survival of breast cancer patients from all subtypes according to CHI3L1 gene expression in primary tumor (n=508, Cutoff--Median), source: E-GEOD-25066. (d) and (e) Relapse free survival of breast cancer patients according to CHI3L1 gene expression in primary tumor (Cutoff--most significant), source: E-GEOD-42568 and GSE29271 respectively.
**Fig 3****:** CHI3L1 inhibits ADCC and Natural cytotoxicity of NK cells and CD8 T cells by impairing lytic granules polarization to the immune synapse. (a) Dose dependent inhibition of NK cell ADCC by rhCHI3L1 against SKBR3 Her2 overexpressing target cells, (b) Effect of 50ng/ml rhCHI3L1 on NK cell ADCC from different healthy donors (N=7). (c) Effect of 50ng/ml rhCHI3L1 on natural cytotoxicity of NK cells against K562 MHC-I missing targets (N«5 healthy donors), (d) Effect of rhCHI3Ll on CD8 T cell cytotoxicity against MEC-1 cells loaded with super-antigen (SEB). (e) Effect of 50ng/ml rhCHI3L1 on murine NK cells ADCC (N=6 mice), (f) Quantification of confocal microscopy images showing frequencies of correctly polarized lytic granules in NK cells conjugated in ADCC with SKBR3 target cells (N=number of NK conjugates counted from 4 healthy donors). All plots are representative of at least two independent evaluations. Values in all graphs represent the means ± SD (95% Cl, *p < 0.05, **p< 0.01, ***p <0.001, NS, not significant).
**Fig 4****:** NK cell ADCC and trastuzumab efficacy is restored by blocking CHI3L1 activity in vitro.
**Fig 5****:** CHI3L1 binds RAGE and blocks its downstream JNK signaling.
   (a) Dot plots for RAGE expression on NK cells (left) and MFI of RAGE expression on NK cells treated with rhCHI3L1. (b) MFI of lytic granules staining in NKs treated with CHI3L1 (50ng/ml) or anti-RAGE Blocking antibody (10ug/ml) for lh. (c) effect of RAGE blockade on NK cells cytotoxicity (d) Basal P-JNK levels in NK cells treated with CHI3L1 (e) P-JNK levels in NK cells after 10 min of stimulation by RAGE ligand S100A8/A9 (10ug/ml) alone or with rhCHI3L1 (100ng/ml) and (f) P-JNK levels in time during ADCC of NK cells incubated with SKBR3 targets at (5:1) E:T ratio. All plots are representative of at least two independent evaluations. Values in all graphs represent the means ± SD (95% Cl, *p < 0.05, **p < 0.01, ***p < 0.001, NS, not significant)

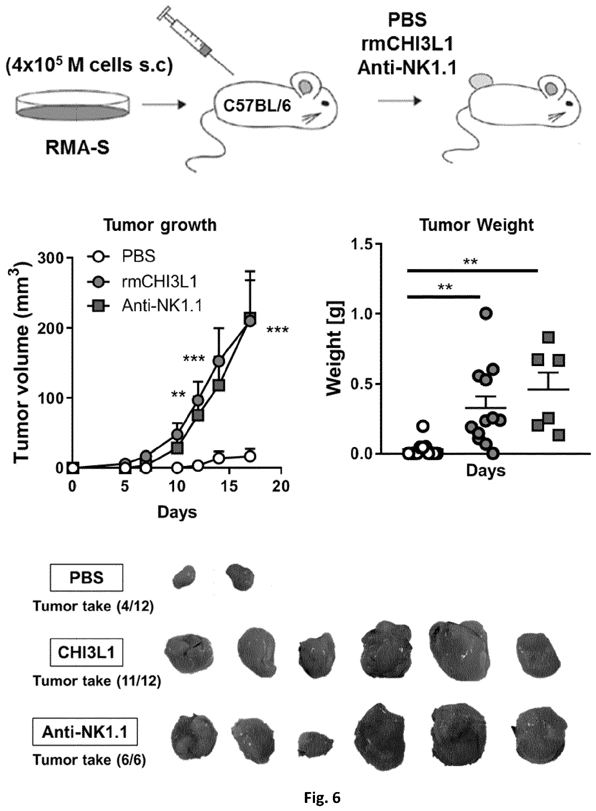

**Fig. 6****: CHI3L1 injections increase tumor take and enhance the growth of NK-sensitive tumors.**
   RMA-S lymphoma cells at limiting number (4×10⁵) were injected s.c in the flank of female C57BL/6 mice at D0. Mice were randomized in three groups to receive PBS, rmCHI3L1 (0.5ug/mouse i.p every other day starting D0), or anti-NK1.1 antibody for NK cell depletion (150ug/mouse i.p on D0, D2, D4). Tumor take and tumor growth were scored every two days. After sacrifice, tumors were weighted and imaged.

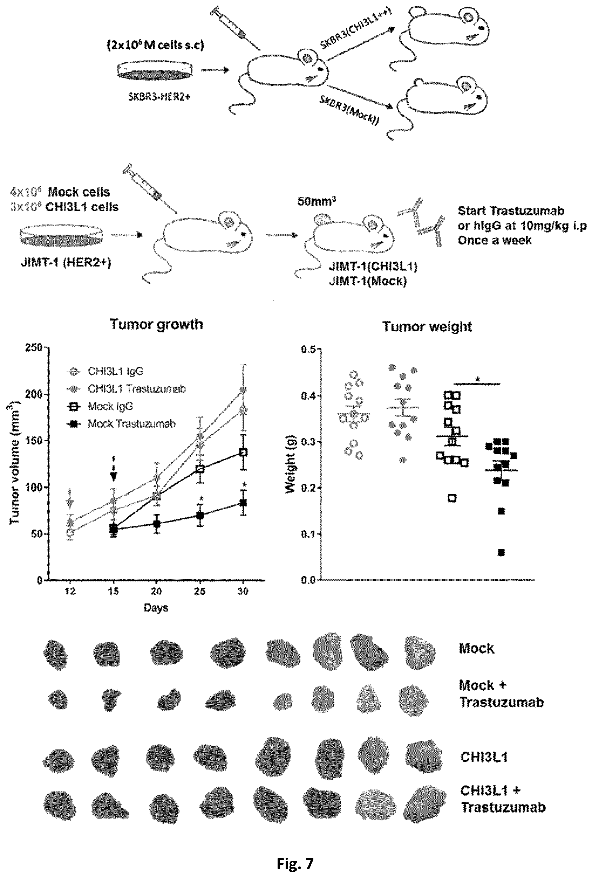

**Figure 7****. CHI3L1 overexpression abrogates the efficacy of Trastuzumab ADCC to control JIMT-1 tumors**
   3×10⁶ JIMT-1 cells overexpressing CHI3L1 (JIMT-1CHI3l1) or 4×10⁶ cells transfected with empty plasmid (JIMT-1 Mock) were injected subcutaneously in the flank of BALB/c Nude mice. The injection of a higher number of JIMT-1 Mock cells was done to compensate for the faster tumor growth of CHI3L1 overexpressing ones. I.p Treatment with 10mg/kg Trastuzumab or hIgG isotype control was started when tumors reached 50mm³ and was given once every week. Tumor take and tumor growth were scored every two days. After sacrifice, tumors were weighted and imaged.

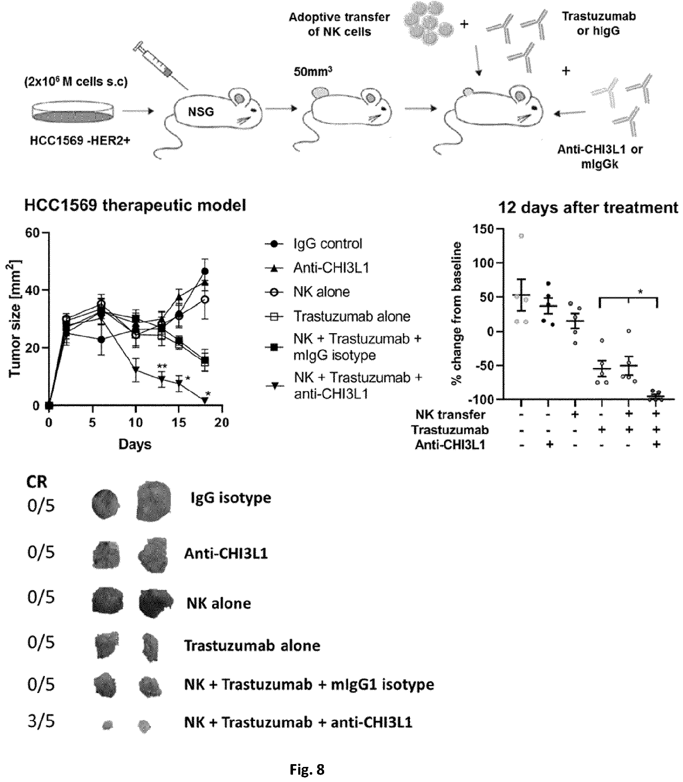

**Figure 8****. Neutralizing CHI3L1 boosts the efficacy of Trastuzumab by unleashing NK cells.** HCC1569 cells (2×10⁶) were injected in the flank of NSG mice. Once tumors reached 50mm³ mice were assigned into the different treatment groups. 8×10⁶ expanded human NK cells were transferred via the tail vein to mice in some groups (D0). Anti-CHI3L1 treatment was started on D-1 (200ug/mouse every two days) and Trastuzumab treatment was started on D1 (200ug/mouse once a week) of NK cell transfer. Experiment was stopped when Isotype control treated mice developed necrotic tumors at which time the Trastuzumab + NK cell + anti-CHI3L1 mice had two tiny Tumors remaining. Mice were then sacrificed and tumors were imaged.

### DETAILED DESCRIPTION OF THE INVENTION

### EXAMPLES

### Example 1

### MATERIALS AND METHODS

### Patients

Women with histological diagnosis of locally advanced invasive or metastatic BC were considered eligible for the study if classified as HER-2 positive, i.e. score 3+ (by immuno-histochemical analysis: IHC) or IHC score 2+ and FISH (fluorescence in situ hybridization) amplified. Thirty BC patients were enrolled in the study that underwent a first line chemotherapy in combination with trastuzumab. Response to trastuzumab was evaluated on the basis of clinical, pathological and radiologic examination of the tumor before and after treatment. The revised RECIST criteria (version 1.1) were used to evaluate the treatment response which was classified as complete response (CR) and disease progression (PD). Blood samples were collected at basal (before Trastuzumab therapy) and subsequently until patients reached PD or RC state (12 months).

This study was approved by the Ethics Committee of the European institute of oncology hospital, Milan, Italy and written informed consent was obtained from each patient.

### Cytotoxicity assay

NK cell and CD8 T cell-mediated cytotoxicity was determined using the DELFIA^{®} EuTDA Cytotoxicity Reagents (PerkinElmer Life Sciences, Waltham, MA, USA), according to manufacturer's instructions. Briefly, target cells (1×10⁶ cells/ml) were incubated with freshly prepared 10 µM BATDA (a fluorescence enhancing ligand) in 2 ml of culture medium for 30 min at 37°C, and washed. Next, 100 µl of BATDA-labeled target cells (5,000 cells) were transferred into a V-bottom sterile plate. In ADCC assays SKBR3 cells (commercial from ATCC, HTB30) were treated with 10ug/ml Trastuzumab (Roche) for 20 min at 37°C and in primary CD8 T cells [from healthy donors, after informed consent] cytotoxicity MEC1 targets (IEO cell line bank) were loaded with 2ug/ml SEB sAg (SIGMA) for 30 min at 37°C. Effector cells were treated as described in each experiment and then co-cultured wit target cells for 1h30min in case of ADCC and 2 hours in case of natural NK cytotoxicity or CD8 T cells cytotoxicity at effector/target ratio of 10: 1 .After incubation, 20 µl of supernatant from each well was transferred to the wells of flat-bottom 96 well plates. 180 µl of europium (Eu) solution was then added to form highly fluorescent and stable chelates (EuTDA), and the fluorescences of these chelates were measured by time-resolved fluorometry (Victor3, PerkinElmer). The percent of specific release was calculated using (experimental release - spontaneous release)/(maximum release - spontaneous release) X 100(%). In blocking experiments, anti-CHI3L1 (clone MaY, MABC196, Sigma-Aldrich) or mIgG1 isotype (EMD millipore) or rhsIL13ra2 (abcam) was pre-incubated with the treatment agent (patient Serum or rhCHI3L1) for 1h before adding the mixture to suspensions of NK cells and incubating for 1h prior to co-cultured with target cells. All experiments were performed in triplicate.

### Flow cytometry

Single-cell suspensions, of blood or from cell culture, were obtained and incubated with Fc receptor-blocking antibody before being stained on ice. Dead cells were excluded by live/dead staining using fixable live/dead stain dyes (Thermofisher scientific). Primary NK cells [from healthy donors, after informed consent] were gated as singlets, CD14neg, FSC-. SSC appropriate for lymphocytes, CD3neg, CD16/CD56poscells. Intracellular staining was performed using a fixation and permeabilization buffer (BD Bioscience). For phosphoflow experiments NK cells were fixed with 4% PFA for 12 min at 37 °C immediately after treatment time and permeabilized with Ice cold PermIII buffer (BDbioscience) for 30 min on ice. Stained cells were analyzed on FACSCanto II cytometer (BD Biosciences). Cell doublets were excluded by comparison of side-scatter width to forward-scatter area. Flow cytometry data were analyzed with FlowJo v10 software (Tree Star).

### RESULTS

To investigate the involvement of NK cell mediated ADCC in trastuzumab resistance, inventors collected sera from metastatic breast cancer patients treated with trastuzumab in adjuvant therapy. NK cells isolated from healthy donors were incubated with the patients' sera and tested for cytotoxic activity. Inventors found that response to trastuzumab can be predicted based on the capacity of serum from treated patients to influence the activity of healthy NK cells where sera from non-responders (N=8) reduced NK cell activity, while sera from responders had no effect on NK cell activity (N=11) (Figure 1).

To identify the molecules involved in the observed phenomenon, inventors carried out a comparative proteomic analysis of the sera of responder versus non-responder patients and identified some molecular candidates that may explain both innate and acquired resistance to trastuzumab. Among these, Chitinase 3-like 1 protein (CHI3L1) was found high at baseline in sera of non-responders or increased during therapy in the sera of patients with acquired resistance (Fig2a). In line with these findings, high CHI3L1 expression in primary tumors correlated with worse overall survival (OS) and progression free survival (PFS) in different breast cancer public cohorts (Fig 2b-e).

To test if CHI3L1 is directly responsible for the decreased cytotoxic activity, we pretreated NK cells with rhCHI3L1 before incubating them with target cells. Inventors found that addition of CHI3L1 impairs both antibody-dependent and natural cytotoxicity of human and murine NK cells in a dose dependent manner (Fig.3a-c,e). Interestingly, inventors observed a similar inhibition of CD8 T cell cytotoxicity against MEC-1 targets loaded with SEB super-antigen (Fig3d). Confocal imaging of the immune synapse between NK cells and target cells revealed an impaired polarization of lytic granules towards the immune synapse in CHI3l1 treated cells (Fig3e)

To test the possibility of restoring trastuzumab dependent cytotoxicity of NK cells, inventors co-incubated with a CHI3L1 neutralizing antibody (mAY) or soluble rhIL13ra2. Both methods abrogated the inhibitory effect of CHI3L1 (Fig. 4).

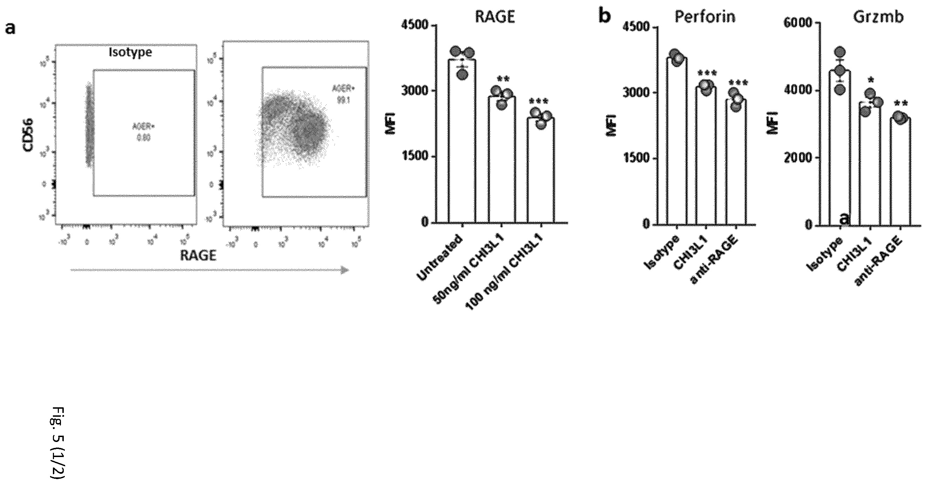

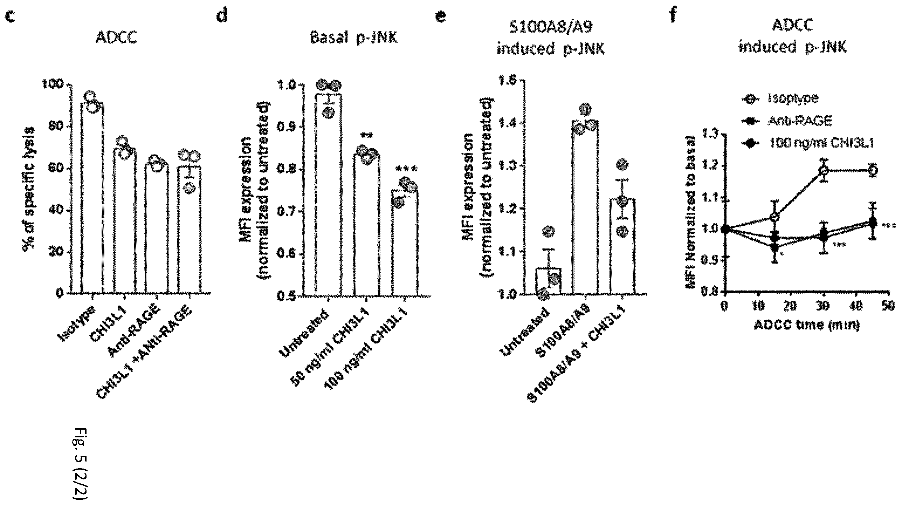

To elucidate the molecular mechanism by which CHI3L1 is acting on NK cells. Inventors first analyzed the expression of receptor of advanced glycation end products (RAGE). Consistent with a previously described activator function of RAGE in murine NK cells inventors found this receptor to be expressed at high levels on CD56+ NK cells. Further, the expression of RAGE was downmodulated after incubation with CHI3L1 suggesting the internalization of RAGE upon CHI3L1 binding (Fig5a).

Inventors next tried to validate the role of RAGE in NK cell cytotoxicity. RAGE blocking antibody, similarly to CHI3L1, inhibited NK cell ADCC and induced a decrease of lytic granules (Fig 5b,c). This suggested to us that CHI3L1 played an inhibitory rather than an activatory function through RAGE binding. To address this issue, inventors evaluated the effect of CHI3L1 ligation on the signaling pathway downstream of RAGE. Inventors focused on pathways that have been shown to be important for NK cell cytotoxicity, including granules biology and polarization, namely ERK1/2 and JNK. While CHI3L1 treatment did not impact on ERK1/2 phosphorylation levels, basal P-JNK levels were decreased in a dose dependent manner in CHI3L1 treated NK cells (Fig4d). Further, CHI3L1 opposed the induction of JNK phosphorylation by the RAGE agonist S100A8/A9, which has been described to activate murine NK cells through RAGE (Fig4e). Inventors found that activation of JNK occurred in a time dependent manner during NK cell ADCC and pretreatment with CHI3L1 or anti-RAGE resulted in a significantly lower P-JNK levels during ADCC suggesting suboptimal JNK phosphorylation in CHI3L1 treated cells (Fig. 5f).

### Example 2

### MATERIALS AND METHODS

### Tumor models

Mice were purchased from Charles River Laboratories and were cared for and used under specific pathogen-free conditions according to the guidelines established in the Principles of Laboratory Animal Care (directive 86/609 /EEC). RMA-S cells were a kind gift from Dr. Sebastian Kobold (LMU, Munich). JIMT-1 and HCC1569 cell lines were purchased from ATCC. Tumor cells at 70% confluence were washed twice and resuspended in cold PBS and kept on ice during the injection procedure. Tumor measurements and endpoints were registered by an observer blinded to the treatment groups.

### RESULTS

RMA-S tumors are vulnerable to NK cell lysis. Injecting mice with CHI3L1 leads to a drastic increase in tumor establishment and tumor growth similar to that of mice where NK cells have been depleted (anti-NK1.1) (Fig. 6). This suggests that CHI3L1 is shutting down NK cell mediated immune surveillance of tumor growth.

JIMT-1 tumors are resistant to Trastuzumab in vitro. However, a partial tumor control can be achieved in vivo thanks to NK cell ADCC. Therefore, a dysfunction of NK cell ADCC in this model will directly reflect negatively on the ability of Trastuzumab to control tumor growth. Indeed, JIMT-1 tumors that do not express CHI3L1 (Mock) were controlled by Trastuzumab injections. On the other hand, CHI3L1 overexpression abrogated tumor control where tumors were rendered completely insensitive to Trastuzumab Injections (Fig. 7). HCC1569 HER2+ BC tumor cells endogenously express high levels of CHI3L1. These cells were derived from a Trastuzumab resistant patient although they still respond partially to Trastuzumab mediated HER2 inhibition in vitro. To closely mimic the human therapeutic setting, we injected these cells in NSG mice and transferred human NK cells to these mice, followed by treatment with Trastuzumab alone, or in combination with anti-human CHI3L1 neutralizing antibody (clone MaY). We observed a partial tumor control by Trastuzumab alone. Importantly NK cell transfer did not have any additional benefit suggesting that NK cells are inhibited in these mice. Only when mice were treated with a CHI3L1 neutralizing antibody, NK cell transfer in combination with Trastuzumab led to complete regression of tumors (Fig. 8). These results demonstrate the therapeutic potential of targeting CHI3L1 to restore NK cell activity and boost Trastuzumab (ADCC) Efficacy in a robust pre-clinical model closely mimicking the human disease.

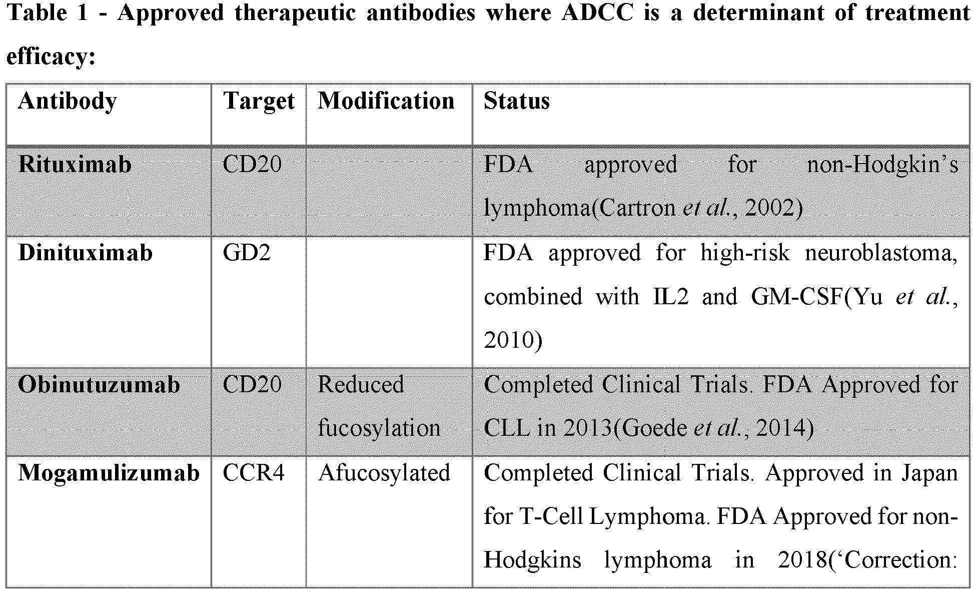

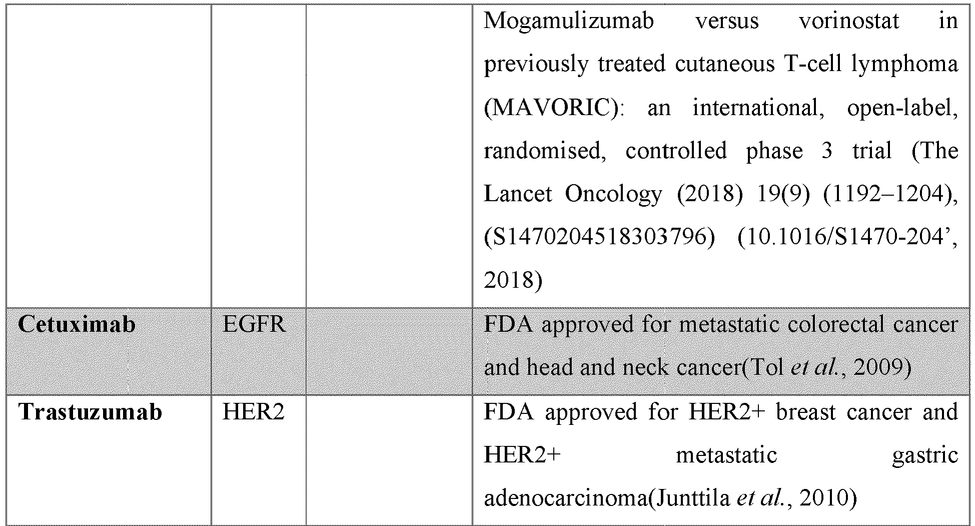

### REFERENCES

1. Cartron, G. et al. Therapeutic activity of humanized anti-CD20 monoclonal antibody and polymorphism in IgG Fc receptor FcyrIIIa gene. Blood 99, 754-758 (2002).
2. Yu, A. L. et al. Anti-GD2 Antibody with GM-CSF, Interleukin-2, and Isotretinoin for Neuroblastoma. N. Engl. J. Med. 363, 1324-1334 (2010).
3. Goede, V. et al. Obinutuzumab plus Chlorambucil in Patients with CLL and Coexisting Conditions. N. Engl. J. Med. 370, 1101-1110 (2014).
4. Correction: Mogamulizumab versus vorinostat in previously treated cutaneous T-cell lymphoma (MAVORIC): an international, open-label, randomised, controlled phase 3 trial (The Lancet Oncology (2018) 19(9) (1192-1204), (S1470204518303796) (10.1016/S1470-204. The Lancet Oncology 19, e581 (2018).
5. Tol, J. et al. Chemotherapy, bevacizumab, and cetuximab in metastatic colorectal cancer.[Erratum appears in N Engl J Med. 2010 Dec 23;363(26):2573]. N. Engl. J. Med. 360, 563-572 (2009).
6. Junttila, T. T. et al. Superior in vivo efficacy of afucosylated trastuzumab in the treatment of HER2-amplified breast cancer. Cancer Res. 70, 4481-4489 (2010).
7. David Zahavi, Dalal AlDeghaither, Allison O'Connell, Louis M Weiner, Enhancing antibody-dependent cell-mediated cytotoxicity: a strategy for improving antibody-based immunotherapy, Antibody Therapeutics, Volume 1, Issue 1, June 2018, Pages 7-12,

## Claims

1. A suppressor or inhibitor of the expression and/or the activity of Chitinase 3-like 1 (CHI3L1) for use in the prevention and/or treatment of tumors,
wherein said tumors are resistant to antibody-dependent cell-mediated cytotoxicity (ADCC) dependent therapies
wherein the tumor comprises HER2 positive cancer cells and is resistant to anti-HER2 antibody therapies, preferably it is resistant to Trastuzumab and/or to Pertuzumab
and wherein the suppressor or inhibitor is at least one molecule selected from the group consisting of: an anti-CHI3L1 antibody or a fragment thereof, and a host cell genetically engineered expressing said antibody or the soluble form of CHI3L1 receptor,
wherein said antibody-dependent cell-mediated cytotoxicity (ADCC) is of Natural Killer (NK) cells.

2. A suppressor or inhibitor of the expression and/or the activity of Chitinase 3-like 1 (CHI3L1) for use in the prevention and/or treatment of tumors,
wherein said tumors are resistant to antibody-dependent cell-mediated cytotoxicity (ADCC) dependent therapies
wherein the tumor is a tumor which is resistant to Trastuzumab
wherein the suppressor or inhibitor is at least one molecule selected from the group consisting of: an anti-CHI3L1 antibody or a fragment thereof, and a host cell genetically engineered expressing said antibody or the soluble form of CHI3L1 receptor,
wherein said antibody-dependent cell-mediated cytotoxicity (ADCC) is of NK cells.

3. The suppressor or inhibitor for use according to claim 1 or 2, wherein the CHI3L1 is extracellular, preferably tumor-derived and/or wherein the tumor expresses CHI3L1 at high levels.

4. The suppressor or inhibitor for use according to any one of previous claims wherein the tumor is a lung, prostate, gastric, colon, rectum, ovary, kidney, liver, breast, glioblastomas, and malignant melanoma .

5. The suppressor or inhibitor for use according to any one of previous claims wherein said suppressor or inhibitor is an anti-CHI3L1 antibody, preferably a monoclonal antibody, more preferably the clone mAY, or an inhibitor or CHI3L1 receptor, or the soluble form of CHI3L1 receptor, preferably interleukin 13 receptor alpha 2 (IL13ra2), or Receptor for Advance Glycation End product (RAGE).

6. The suppressor or inhibitor for use according to claim 5, wherein the tumor is resistant to Trastuzumab.

7. The suppressor or inhibitor for use according to any one of previous claims, used in combination with at least one therapeutic antibody which is capable of antibody-dependent cell-mediated cytotoxicity (ADCC), preferably an anti-HER2, an anti-CD20, an anti-GD20, an anti-CCR4, an anti-EGFR, an anti-CD19 or an anti-MUC1 or an anti-GA201 antibody, more preferably with Trastuzumab, Pertuzumab, Rituximab, Dinituximab, Obinutuzumab, Mogamulizumab, Panitumumab and/or Cetuximab, more preferably Trastuzumab and/or Pertuzumab.

8. A pharmaceutical composition comprising:
i. at least one suppressor or inhibitor of expression and/or activity of Chitinase 3-like 1 (CHI3L1) as defined in any one of claims 1-7,
ii. at least one therapeutic antibody which is capable of antibody-dependent cell-mediated cytotoxicity (ADCC) and
iii. at least one pharmaceutically acceptable excipient, diluent or carrier for use in the prevention and/or treatment of tumors,
wherein said tumors are resistant to antibody-dependent cell-mediated cytotoxicity (ADCC) dependent therapies, wherein the tumor comprises HER2 positive cancer cells, preferably a breast tumor or a gastric cancer, and is resistant to anti-HER2 antibody therapies, preferably it is resistant to Trastuzumab and/or to Pertuzumab.
preferably said antibody which is capable of antibody-dependent cell-mediated cytotoxicity (ADCC) is at least one antibody anti-HER2, an anti-CD20, an anti-GD20, an anti-CCR4, an anti-EGFR, an anti-CD19 or an anti-MUC1 or an anti-GA201, more preferably it is at least one antibody selected from the group consisting of: Trastuzumab, Pertuzumab, Rituximab, Dinituximab, Obinutuzumab, Mogamulizumab, Panitumumab and/or Cetuximab, more preferably Trastuzumab and/or Pertuzumab.

9. The pharmaceutical composition of claim 8 further comprising a therapeutic agent, wherein said therapeutic agent is for the treatment and/or prevention of tumours.

## Patentansprüche

1. Suppressor oder Inhibitor der Expression und/oder der Aktivität von Chitinase 34-like 1 (CHI3L1) zur Verwendung bei der Prävention und/oder Behandlung von Tumoren,
wobei diese Tumore gegen Therapien resistent sind, die antikörperabhängige zellvermittelte Zytotoxizität (ADCC) voraussetzen,
wobei der Tumor HER2-positive Krebszellen umfasst und gegen Anti-HER2-Antikörper-Therapien resistent ist, vorzugsweise ist er resistent gegen Trastuzumab und/oder gegen Pertuzumab,
und wobei der Suppressor oder Inhibitor mindestens ein Molekül ist, ausgewählt aus der Gruppe bestehend aus: einem Anti-CH13L1-Antikörper oder einem Fragment davon und einer Wirtszelle, die gentechnisch verändert wurde und den Antikörper oder die lösliche Form des CH13L1-Rezeptors exprimiert,
wobei die antikörperabhängige zellvermittelte Zytotoxizität (ADCC) von natürlichen Killerzellen (NK-Zellen) ausgeht.

2. Suppressor oder Inhibitor der Expression und/oder der Aktivität von Chitinase 3-like 1 (CH13L1) zur Verwendung bei der Prävention und/oder Behandlung von Tumoren,
wobei die Tumore gegen Therapien resistent sind, die antikörperabhängige zellvermittelte Zytotoxizität (ADCC) voraussetzen,
wobei der Tumor ein Tumor ist, der gegen Trastuzumab resistent ist,
wobei der Suppressor oder Inhibitor mindestens ein Molekül ist, ausgewählt aus der Gruppe bestehend aus: einem Anti-CH13L1-Antikörper oder einem Fragment davon und einer Wirtszelle, die gentechnisch verändert wurde und den Antikörper oder die lösliche Form des CH13L1-Rezeptors exprimiert,
wobei die antikörperabhängige zellvermittelte Zytotoxizität (ADCC) von NK-Zellen ausgeht.

3. Suppressor oder Inhibitor zur Verwendung nach Anspruch 1 oder 2, wobei das CH13L1 extrazellulär ist, vorzugsweise aus einem Tumor stammt und/oder wobei der Tumor CH13L1 in hohen Mengen exprimiert.

4. Suppressor oder Inhibitor zur Verwendung nach einem der vorhergehenden Ansprüche, wobei es sich bei dem Tumor um Lunge, Prostata, Magen, Kolon, Rektum, Eierstock, Niere, Leber, Brust, Glioblastome und malignes Melanom handelt.

5. Suppressor oder Inhibitor zur Verwendung nach einem der vorhergehenden Ansprüche, wobei der Suppressor oder Inhibitor ein Anti-CH13L1-Antikörper, vorzugsweise ein monoklonaler Antikörper, besonders bevorzugt der Klon mAY, oder ein Inhibitor oder CH13L1-Rezeptor oder die lösliche Form des CH13L1-Rezeptors, vorzugsweise Interleukin-13-Rezeptor alpha 2 (IL13ra2) oder Receptor for Advance Glycation End product (RAGE) ist.

6. Suppressor oder Inhibitor zur Verwendung nach Anspruch 5, wobei der Tumor gegen Trastuzumab resistent ist.

7. Suppressor oder Inhibitor zur Verwendung nach einem der vorhergehenden Ansprüche, verwendet in Kombination mit mindestens einem therapeutischen Antikörper, der zu antikörperabhängiger zellvermittelter Zytotoxizität (ADCC) fähig ist, vorzugsweise einem Anti-HER2, einem Anti-CD20, einem Anti-GD20, einem Anti-CCR4-, einem Anti-EGFR-, einem Anti-CD19- oder einem Anti-MUC1- oder einem Anti-GA201-Antikörper, besonders bevorzugt mit Trastuzumab, Penuzumab, Rituximab, Dinituximab, Obinutuzumab, Mogamulizumab, Panitumumab und/oder Cetuximab, besonders bevorzugt mit Trastuzumab und/oder Pertuzumab.

8. Pharmazeutische Zusammensetzung, umfassend:
i. mindestens einen Suppressor oder Inhibitor der Expression und/oder Aktivität von Chitinase 3-like 1 (CH13L1), wie in einem der Ansprüche 1-7 definiert,
ii. mindestens einen therapeutischen Antikörper, der zu antikörperabhängiger zellvermittelter Zytotoxizität (ADCC) fähig ist, und
iii. mindestens einen pharmazeutisch akzeptablen Hilfsstoff, Verdünner oder Träger zur Verwendung bei der Prävention und/oder Behandlung von Tumoren,
wobei die Tumore resistent sind gegen Therapien, die antikörperabhängige zellvermittelte Zytotoxizität (ADCC) voraussetzen, wobei der Tumor HER2-positive Krebszellen umfasst, vorzugsweise einen Brusttumor oder einen Magenkrebs, und resistent ist gegen Anti-HER2-Antikörper-Therapien, vorzugsweise ist er resistent gegen Trastuzumab und/oder gegen Pertuzumab, vorzugsweise handelt es sich bei dem Antikörper, der zur antikörperabhängigen zellvermittelten Zytotoxizität (ADCC) fähig ist, um mindestens einen Anti-HER2-Antikörper, einen Anti-CD20-Antikörper, einen Anti-GD20-Antikörper, einen Anti-CCR4-Antikörper, einen Anti-EGFR-Antikörper, einen Anti-CD19-Antikörper oder einen Anti-MUC1-Antikörper oder einen Anti-GA201-Antikörper, wobei es sich vorzugsweise um mindestens einen Antikörper handelt, ausgewählt aus der Gruppe bestehend aus: Trastuzumab, Pertuzumab, Rituximab, Dinituximab, Obinutuzumab, Mogamulizumab, Panitumumab und/oder Cetuximab, besonders bevorzugt Trastuzumab und/oder Pertuzumab.

9. Pharmazeutische Zusammensetzung nach Anspruch 8, ferner umfassend einen therapeutischen Wirkstoff, wobei der therapeutische Wirkstoff zur Behandlung und/oder Prävention von Tumoren dient.

## Revendications

1. Suppresseur ou inhibiteur de l'expression et/ou de l'activité de la chitinase 3-like 1 (CHI3L1) destiné à être utilisé dans la prévention et/ou le traitement de tumeurs,
dans lequel lesdites tumeurs sont résistantes aux thérapies dépendantes de la cytotoxicité à médiation cellulaire dépendante des anticorps (ADCC)
dans lequel la tumeur comprend des cellules cancéreuses HER2 positives et est résistante aux thérapies par anticorps anti-HER2, de préférence elle est résistante au trastuzumab et/ou au pertuzumab
et dans lequel le suppresseur ou l'inhibiteur est au moins une molécule sélectionnée dans le groupe constitué par : un anticorps anti-CHI3L1 ou un fragment de celui-ci, et une cellule hôte génétiquement modifiée exprimant ledit anticorps ou la forme soluble du récepteur de CHI3L1,
dans lequel ladite cytotoxicité à médiation cellulaire dépendante des anticorps (ADCC) est des cellules tueuses naturelles (NK).

2. Suppresseur ou inhibiteur de l'expression et/ou de l'activité de la chitinase 3-like 1 (CHI3L1) destiné à être utilisé dans la prévention et/ou le traitement de tumeurs,
dans lequel lesdites tumeurs sont résistantes aux thérapies dépendantes de la cytotoxicité à médiation cellulaire dépendante des anticorps (ADCC)
dans lequel la tumeur est une tumeur qui est résistante au trastuzumab
dans lequel le suppresseur ou l'inhibiteur est au moins une molécule sélectionnée dans le groupe constitué par : un anticorps anti-CHI3L1 ou un fragment de celui-ci, et une cellule hôte génétiquement modifiée exprimant ledit anticorps ou la forme soluble du récepteur CHI3L1,
dans lequel ladite cytotoxicité à médiation cellulaire dépendante des anticorps (ADCC) est des cellules NK.

3. Suppresseur ou inhibiteur destiné à être utilisé selon la revendication 1 ou 2, dans lequel la CHI3L1 est extracellulaire, de préférence dérivée d'une tumeur et/ou dans lequel la tumeur exprime CHI3L1 à des niveaux élevés.

4. Suppresseur ou inhibiteur destiné à être utilisé selon l'une quelconque des revendications précédentes dans lequel la tumeur est un cancer du poumon, de la prostate, gastrique, du côlon, du rectum, de l'ovaire, du rein, du foie, du sein, des glioblastomes et un mélanome malin.

5. Suppresseur ou inhibiteur destiné à être utilisé selon l'une quelconque des revendications précédentes dans lequel ledit suppresseur ou inhibiteur est un anticorps anti-CHI3Ll, de préférence un anticorps monoclonal, plus préférablement le clone mAY, ou un inhibiteur ou un récepteur CHI3L1, ou la forme soluble du récepteur CHI3L1, de préférence le récepteur alpha 2 de l'interleukine 13 (IL13ra2), ou un récepteur pour le produit final de glycation avancée (RAGE).

6. Suppresseur ou inhibiteur destiné à être utilisé selon la revendication 5, dans lequel la tumeur est résistante au trastuzumab.

7. Suppresseur ou inhibiteur destiné à être utilisé selon l'une quelconque des revendications précédentes, utilisé en association avec au moins un anticorps thérapeutique qui est capable de cytotoxicité à médiation cellulaire dépendante des anticorps (ADCC), de préférence un anti-HER2, un anti-CD20, un anti-GD20, un anti-CCR4, un anti-EGFR, un anti-CD19 ou un anti-MUC1 ou un anticorps anti-GA201, plus préférablement avec le trastuzumab, le pertuzumab, le rituximab, le dinituximab, l'obinutuzumab, le mogamulizumab, le panitumumab et/ou le cétuximab, plus préférablement le trastuzumab et/ou le pertuzumab.

8. Composition pharmaceutique comprenant :
i. au moins un suppresseur ou inhibiteur de l'expression et/ou de l'activité de la chitinase 3-like 1 (CHI3L1) tel que défini dans l'une quelconque des revendications 1 à 7,
ii. au moins un anticorps thérapeutique qui est capable d'une cytotoxicité à médiation cellulaire dépendante des anticorps (ADCC) et
iii. au moins un excipient, diluant ou véhicule pharmaceutiquement acceptable
destinée à être utilisée dans la prévention et/ou le traitement de tumeurs, dans laquelle lesdites tumeurs sont résistantes aux thérapies dépendantes de la cytotoxicité à médiation cellulaire dépendante des anticorps (ADCC), dans laquelle la tumeur comprend des cellules cancéreuses HER2 positives, de préférence une tumeur du sein ou un cancer gastrique, et est résistante aux thérapies par anticorps anti-HER2, de préférence elle est résistante au trastuzumab et/ou au pertuzumab de préférence ledit anticorps qui est capable de cytotoxicité à médiation cellulaire dépendante des anticorps (ADCC) est au moins un anticorps anti-HER2, un anti-CD20, un anti-GD20, un anti-CCR4, un anti-EGFR, un anti-CD19 ou un anti-MUC1 ou un anticorps anti-GA201, plus préférablement c'est au moins un anticorps sélectionné dans le groupe constitué par : le trastuzumab, le pertuzumab, le rituximab, le dinituximab, l'obinutuzumab, le mogamulizumab, le panitumumab et/ou le cétuximab, plus préférablement le trastuzumab et/ou le pertuzumab.

9. Composition pharmaceutique selon la revendication 8 comprenant en outre un agent thérapeutique, dans laquelle ledit agent thérapeutique est destiné au traitement et/ou à la prévention de tumeurs.
